# EUROPEAN PATENT SPECIFICATION

(11) **EP 2 858 490 B1**
(45) Date of publication and mention of the grant of the patent: **26.10.2016**
(21) Application number: 12753914.6
(22) Date of filing: 12.06.2012
(51) Int. Cl.: C12N 15/86, A01K 67/033, C12N 15/85, C12N 15/866

(54) **RECOMBINANT DNA ELEMENTS FOR THE EXPRESSION OF RECOMBINANT PROTEINS IN INSECTS**
REKOMBINANTE DNS-ELEMENTE ZUR EXPRESSION REKOMBINANTER PROTEINE IN INSEKTEN
ÉLÉMENTS ADN RECOMBINANTS POUR L'EXPRESSION DE PROTÉINES RECOMBINANTES CHEZ DES INSECTES

(43) Date of publication of application: 15.04.2015
(73) Proprietor: Alternative Gene Expression S.L., 28223 Pozuelo de Alarcón, Madrid (ES)
(72) Inventor: GOMEZ SEBASTIAN, Silvia, E-28040 Madrid (ES); LÓPEZ VIDAL, Javier, Edinburgh EH9 3FL (GB); MARTINEZ ESCRIBANO, José Angel, E-28040 Madrid (ES)
(74) Representative: Hoffmann Eitle
(86) International application number: PCT/EP2012/061088
(87) International publication number: WO 2012/168493

(56) References cited:
- EP-A1- 1 811 027
- WO-A1-2010/025764
- US-A1- 2009 068 703
- NAGAI S ET AL: "Comparative transient expression assay analysis of hycu-hr6- and IE1-dependent regulation of baculovirus gp64 early promoters in three insect cell lines", VIRUS RESEARCH, AMSTERDAM, NL, vol. 155, no. 1, 1 January 2011 (2011-01-01), pages 83-90, XP027574274, ISSN: 0168-1702 [retrieved on 2010-12-24]
- CROUCH E A ET AL: "Effects of baculovirus transactivators IE-1 and IE-2 on the Drosophila heat shock 70 promoter in two insect cell lines", ARCHIVES OF VIROLOGY ; OFFICIAL JOURNAL OF THE VIROLOGY DIVISIONOF THE INTERNATIONAL UNION OF MICROBIOLOGICAL SOCIETIES, SPRINGER-VERLAG, VI, vol. 150, no. 8, 1 August 2005 (2005-08-01), pages 1563-1578, XP019378571, ISSN: 1432-8798, DOI: 10.1007/S00705-005-0527-8
- NAGAMINE T ET AL: "Induction of a subnuclear structure by the simultaneous expression of baculovirus proteins, IE1, LEF3, and P143 in the presence of hr", VIROLOGY, ACADEMIC PRESS,ORLANDO, US, vol. 352, no. 2, 1 September 2006 (2006-09-01), pages 400-407, XP024896408, ISSN: 0042-6822, DOI: 10.1016/J.VIROL.2006.04.034 [retrieved on 2006-09-01]
- W. KANG: "IE1 and hr facilitate the localization of Bombyx mori nucleopolyhedrovirus ORF8 to specific nuclear sites", JOURNAL OF GENERAL VIROLOGY, vol. 86, no. 11, 1 November 2005 (2005-11-01), pages 3031-3038, XP055016117, ISSN: 0022-1317, DOI: 10.1099/vir.0.81270-0
- LIN X; CHEN Y; YI Y; ZHANG Z: "Baculovirus immediately early 1, a mediator for homologous regions enhancer function in trans", VIROL J, vol. 7, no. 32, 2010, XP002685888, cited in the application
- PASSARELLI ET AL: "Three baculovirus genes involved in late and very late gene expression: ie-1, ie-n, and lef-2.", JOURNAL OF VIROLOGY, vol. 67, no. 4, 1 April 1993 (1993-04-01), pages 2149-2158, XP055016120, ISSN: 0022-538X cited in the application
- NETTLESHIP J E ET AL: "Recent advances in the production of proteins in insect and mammalian cells for structural biology", JOURNAL OF STRUCTURAL BIOLOGY, ACADEMIC PRESS, UNITED STATES, vol. 172, no. 1, 1 October 2010 (2010-10-01), pages 55-65, XP027249557, ISSN: 1047-8477 [retrieved on 2010-02-11] cited in the application
- S. KANGINAKUDRU ET AL: "Targeting ie-1 gene by RNAi induces baculoviral resistance in lepidopteran cell lines and in transgenic silkworms", INSECT MOLECULAR BIOLOGY, vol. 16, no. 5, 1 October 2007 (2007-10-01), pages 635-644, XP055029022, ISSN: 0962-1075, DOI: 10.1111/j.1365-2583.2007.00753.x
- V. J. VALDES ET AL: "Using Double-stranded RNA to Prevent in Vitro and in Vivo Viral Infections by Recombinant Baculovirus", JOURNAL OF BIOLOGICAL CHEMISTRY, vol. 278, no. 21, 16 May 2003 (2003-05-16) , pages 19317-19324, XP055029633, ISSN: 0021-9258, DOI: 10.1074/jbc.M212039200
- OGAWA ET AL: "Generation of a transgenic silkworm that secretes recombinant proteins in the sericin layer of cocoon: Production of recombinant human serum albumin", JOURNAL OF BIOTECHNOLOGY, ELSEVIER SCIENCE PUBLISHERS, AMSTERDAM, NL, vol. 128, no. 3, 24 January 2007 (2007-01-24), pages 531-544, XP005856750, ISSN: 0168-1656, DOI: 10.1016/J.JBIOTEC.2006.10.019
- MASAHIRO TOMITA ET AL: "A germline transgenic silkworm that secretes recombinant proteins in the sericin layer of cocoon", TRANSGENIC RESEARCH, KLUWER ACADEMIC PUBLISHERS-PLENUM PUBLISHERS, NE, vol. 16, no. 4, 6 April 2007 (2007-04-06), pages 449-465, XP019534548, ISSN: 1573-9368, DOI: 10.1007/S11248-007-9087-X
- GOMI S, MAJIMA K, MAEDA S.: "Sequence analysis of the genome of Bombyx mori nucleopolyhedrovirus.", J GEN VIROL., vol. 80, no. 5, May 1999 (1999-05), pages 1323-1337, XP002686324,
- BETAPUDI VENKAIAH ET AL: "An Additional Copy of the Homologous Region (hr1) Sequence in the Autographa californica Multinucleocapsid Polyhedrosis Virus Genome Promotes Hyperexpression of Foreign Genes +", BIOCHEMISTRY, vol. 43, no. 25, 1 June 2004 (2004-06-01), pages 8143-8151, XP055042767, ISSN: 0006-2960, DOI: 10.1021/bi049953q
- MAJIMA ET AL: "Divergence and evolution of homologous regions of Bombyx mori nuclear polyhedrosis virus.", JOURNAL OF VIROLOGY, vol. 67, no. 12, 1 December 1993 (1993-12-01), pages 7513-7521, XP055042758, ISSN: 0022-538X
- GOMEZ-CASADO E; GOMEZ-SEBASTIAN S; NÚÑEZ MC; LASA-COVARRUBIAS R; MARTÍNEZ-PULGARÍN S; ESCRIBANO JM: "Insect larvae biofactories as a platform for influenza vaccine production", PROTEIN EXPR PURIF., vol. 79, 2011, pages 35-43, XP002685889,

## Description

### FIELD OF THE INVENTION

The present invention may be included in the field of biotechnology and it covers insects comprising nucleic acid sequences comprising, for example, promoters, homologous regions (*hr*) as enhancers, and sequences encoding transcriptional regulators, for example, the baculovirus *Ac-ie-01* cDNA, or any combination thereof, which are able to increase the efficiency of recombinant protein expression, for example in insect larvae. Moreover, the present invention is also directed to methods using the above mentioned insect of the invention infected, transformed, transduced or transfected with those sequences or vectors, and methods for producing proteins by using the aforesaid insects.

### STATE OF THE ART

The baculovirus expression vector system (BEVS) is a well-established method for the production of recombinant proteins to be used as vaccines, therapeutic molecules or diagnostic reagents. With its potential for over-expression and rapid speed of development, BEVS is one of the most attractive choices for producing recombinant proteins for any purpose. The most employed baculovirus vector used in industry for recombinant protein expression is based on *Autographa californica* multinuclear polyhedrosis virus (*Ac*MNPV) with *Spodoptera frugiperda* 9 (*Sf*9) or 21 (*Sf*21) insect cells as suitable expression hosts (1), as well as *Trichoplusia ni* (*T*. *ni*) insect larvae as living biofactories (2). Since the BEVS was developed in the 80's (3), hundreds of recombinant proteins, ranging from cytosolic enzymes to membrane-bound proteins, have been successfully produced in baculovirus-infected insect cells.

Worldwide, about 70,000 tons of silk are produced annually in a process that converts a low-value substrate, the leaves of the mulberry tree, to a high-value protein-based product: silk. Insects are highly efficacious protein producers because of their accelerated metabolism. Lepidoptera such as *Bombyx mori* or *Trichoplusia ni,* are two of the most used insects in biotechnology. They grow in size about 5000 times in less than 2 weeks and produce more than a kilometer of silk per insect. While a cell from a silk gland may produce about 80 µg protein/cell/day, the best mammalian cell culture systems produces only about 50 pg protein/cell/day. Concerns arising from potential contamination by adventitious agents (such as viruses or prions) of recombinant proteins obtained from cultured mammalian cells are substantially lower, if not totally absent, in the case of insect-derived products for human or animal use. Although transgenic silkworms have been generated to produce human therapeutic proteins (4), this technology has not yet been used to produce vaccine antigens. However, genetically modified baculoviruses (*Autographa californica* nuclear polyhedrosis virus and *Bombyx mori* nucleopolyhedrovirus) have been used to generate insect-derived vaccines (insectigens). Basically, the same baculoviruses used for antigen expression in insect cell cultures can be used to infect insect larvae. Upon infection, the recombinant antigen is accumulated in insect tissues and, after 3-4 days of infection, the larvae can be processed to obtain the recombinant antigen in quantities that can reach between several hundreds of µg to several milligrams per infected larva. Experimental vaccines against animal infectious diseases have been tested with very good results, even using nonpurified soluble antigens obtained from larva, without any side effects in animals after repetitive immunization protocols (5, 6, 7, 8). Other proteins with different applications have also been produced in insects as living biofactories, such as enzymes (9, 10), antibodies (11, 12), hormones (13, 14), cytokines (15, 16) and diagnostic proteins (17, 18, 19, 20). Most of these above mentioned proteins were processed correctly after synthesis, and their functional activities remained intact in soluble larval protein extracts. Insects as living biofactories constitute a promising alternative to conventional fermentation technologies and also to plant-derived proteins because of the production versatility, scalability, efficiency and speed of development.

Accelerating recombinant protein expression, so that protein expression takes place before the cellular machinery of insect larvae is severely impaired by the baculovirus infection, would be an important improvement of the BEVS. Late expression, driven by the conventional strong virus promoters of *polyhedrin (polh)* or *p10* genes, has serious disadvantages in the foreign protein post-translational modifications. Baculovirus promoters that allow for earlier expression than the conventionally used *polh* or *p10* promoters have been characterized and been used for heterologous protein production, but showed a reduced productivity (21).

Another possibility for improving the BEVS would be to increase preservation of cell integrity at late times post-infection by reducing the virus-induced cell death. Reduction in the severe impairment of the insect cell machinery at late times post-infection caused by BEVS should not only increase the time frame for producing and accumulating recombinant proteins (secreted or not), but also allow more time for the folding of complex proteins or any post-translational modification of the produced proteins. Baculovirus-infected insect larvae produce and accumulate recombinant proteins during the course of infection and in a virus dose-dependent manner. A prolonged survival of the infected larvae together with a certain protection to high infectious doses would dramatically increase the productivity of insects serving as living biofactories.

Some baculovirus DNA elements have been determined to be involved in the activation of late expression factor genes, which are necessary for virus propagation. One of them is the immediate early (ie) protein IE-1 and its splice variant IE-0 from *Ac*MNPV (*Autographa californica* multinuclear polyhedrosis virus). Translation of the *Ac*MNPV mRNAs encoded by *Ac-ie-01* cDNA results in both IE-0 and IE-1 production due to internal translation initiation. Both are thought to be critical mediators of baculovirus gene expression due to their potency as transcriptional regulators (22). Synthesized very early during infection, *Ac*MNPV IE-1 is a 67-kDa dimeric DNA-binding protein that stimulates transcription in plasmid transfection assays through the activity of its N-terminal acidic domain (23, 24). IE-1 accumulates within the nucleus, where it is maintained through late times (25). Transactivation by IE-1 is enhanced by its binding as a homodimer to the baculovirus homologous region *(hr)* sequences, which function as transcriptional enhancers and origins of viral DNA replication. *Ac*MNPV immediate early protein IE-0 (74 kDa) is identical to IE-1 except for an additional 54 amino acid residues at its N-terminus. *Ac*MNPV IE-0 is a 72.6-kDa 636 amino acid protein composed of 38 amino acids encoded by *orf141* (*exon0*)*,* 16 amino acids encoded by the upstream nontranslated leader of *ie1,* and the entire 582 amino acid IE-1 protein. The final product is therefore identical to IE-1 except for the additional 54 amino acids fused to the N-terminus. Presumably due to their common sequences, IE-0 and IE-1 share biochemical activities, including hr enhancer binding and transcriptional regulation.

Given the lack of novel alternative promoters stronger than those commercially used (*polh* and *p10*) and of any alternative to implement long-term expression in the baculovirus system by reducing virus-induced cell damage, the present invention is focused on solving said problems by means of the incorporation of recombinant DNA elements in baculovirus expression cassettes. The present invention surprisingly shows that said expression cassettes, containing baculovirus transcriptional regulators, an enhancer homologous region (hr) sequence and a promoter or a combination of promoters are able to increase recombinant protein production to unprecedented levels in insect larvae. Additionally, the expression cassettes as described also increase the survival rate of baculovirus-infected insect larvae at late times post-infection using high infectious doses, thus minimizing the pathogenicity effect in insects caused by the baculovirus infection. On the other hand, an improvement in the integrity of cell functions during baculovirus infection also contributes to the correct recombinant protein posttranslational processing in insect larvae.

V.J. Valdes et al. disclose the use of double-stranded RNA to prevent in vitro and in vivo viral infections by recombinant baculovirus (V.J.Valdes et al., Journal of Biological Chemistry, Vol. 278, No. 21 16 May 2003, pages 19317 - 19234). EP1 811 027 discloses polynucleotides for the production of recombinant proteins in silkworm.

### SUMMARY OF THE INVENTION

The present invention provides products and methods for the improved expression of recombinant proteins in baculovirus-infected insect larvae, as also defined in the claims:
1. Insect derived from the genus Lepidoptera, comprising nucleic acid elements introduced into the insect by a recombinant baculovirus containing an endogenous *Ac-ie-01* gene, wherein said nucleic acid elements comprise
   (a) a further copy of baculoviral *Ac-ie-01* cDNA as transgene under the control of a suitable promoter that allows for the expression of the proteins IE-1, IE-0 and/or fragments thereof functioning as transcriptional regulators above endogenous levels obtained during baculovirus infection, wherein the nucleic acid is selected from the group consisting of:
      i. a nucleic acid containing the nucleotide sequence indicated in any of SEQ ID NO: 1-5;
      ii. a nucleic acid sequence having a sequence identity of at least 70%, preferably at least 75%, more preferably at least 80%, more preferably at least 85%, more preferably at least 90% and most preferably at least 95% with the nucleotide sequence indicated in any of SEQ ID NO: 1-5 and encoding a protein able to function as a transcriptional regulator in a recombinant baculovirus;
      iii. a nucleic acid sequence encoding an amino acid containing the amino acid sequence indicated in any of SEQ ID NO: 6-9; and
      iv. a nucleic acid sequence encoding an amino acid sequence having a sequence similarity of at least 70%, preferably at least 75%, more preferably at least 80%, more preferably at least 85%, more preferably at least 90% and most preferably at least 95% with the amino acid sequence indicated in any of SEQ ID NO: 6-9 and able to function as a transcriptional regulator in a recombinant baculovirus;
   (b) wherein the insect further comprises at least one recombinant homologous region (*hr*) as enhancer region, operably linked to any promoter that is suitable for driving the expression of a recombinant protein,
   (c) wherein the promoter that drives the expression of said recombinant protein is selected from the group of nucleic acids comprising:
      i. a nucleic acid containing the nucleotide sequence indicated in any of SEQ ID NO: 10-16; and
      ii. a nucleic acid sequence able to function as a promoter in a recombinant baculovirus and having a sequence identity of at least 70%, preferably at least 75%, more preferably at least 80%, more preferably at least 85%, more preferably at least 90% and most preferably at least 95% with the nucleotide sequence indicated in any of SEQ ID NO: 10-16.
2. Insect according to item 1, wherein the recombinant homologous region *(hr)* is the sequence indicated in SEQ ID NO: 27 (*hr1*)*.*
3. Insect according to any of the items 1-2, wherein the nucleic acid sequence that comprises combinations of recombinant promoters, sequences encoding transcriptional regulators and enhancer regions are selected from the group comprising SEQ ID NO: 17-26.
4. Insect according to any of the items 1-3, wherein the insect is selected from the group consisting of *Trichoplusia ni, Spodoptera frugiperda, Spodoptera exigua, Ascalapha odorata, Bombyx mori, Rachiplusia ni and Estigmene acrea.*
5. Insect according to any of the items 1-4, wherein the nucleic acid sequence(s) is introduced into the insect by a recombinant baculovirus selected from *Ac*MNPV or *Bm*NPV.
6. Insect according to any of the items 1-5, further comprising a nucleic acid sequence encoding a recombinant protein, wherein said nucleic acid sequence is operably linked to a nucleic acid sequence selected from the group consisting of the nucleic acid sequences of items 1-3.
7. Method for producing a recombinant protein that comprises the use of an insect of item 6 and the extraction and purification of the recombinant protein by conventional means.
8. Method for producing a recombinant protein according to item 7, wherein the recombinant protein is selected from the group comprising subunit monomeric vaccine, subunit multimeric vaccine, virus like particle, therapeutic protein, antibody, enzyme, cytokine, blood clotting factor, anticoagulant, receptor, hormone or diagnostic protein reagent.

### BRIEF DESCRIPTION OF THE FIGURES

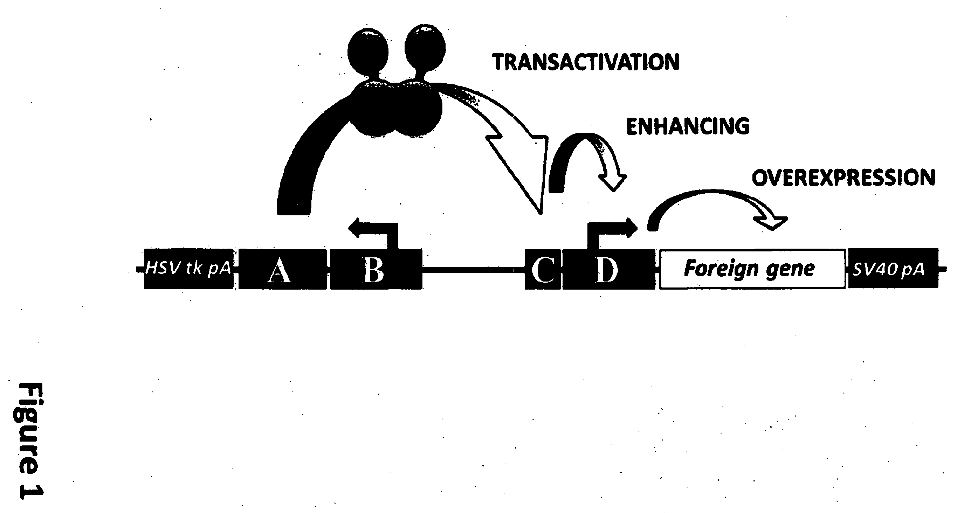

**Figure 1****:** Schematic representation of the baculovirus recombinant DNA elements of the invention, comprising four principal elements: a sequence encoding for transcriptional regulators (**A**; e.g. IE0 and IE1), which expression is driven by a promoter (**B**; e.g. *polh or pB29*); an enhancer homologous region (*hr*) sequence (**C**; e.g. *hr1*), upstream of the promoters (**D**; e.g. *p10, pB29p10* or *p6.9*p10) driving the expression of the foreign gene coding for the recombinant protein. The scheme shows the theoretical mechanism of interaction between the recombinant DNA elements of the present invention that results in the unprecedented overexpression of the recombinant protein.

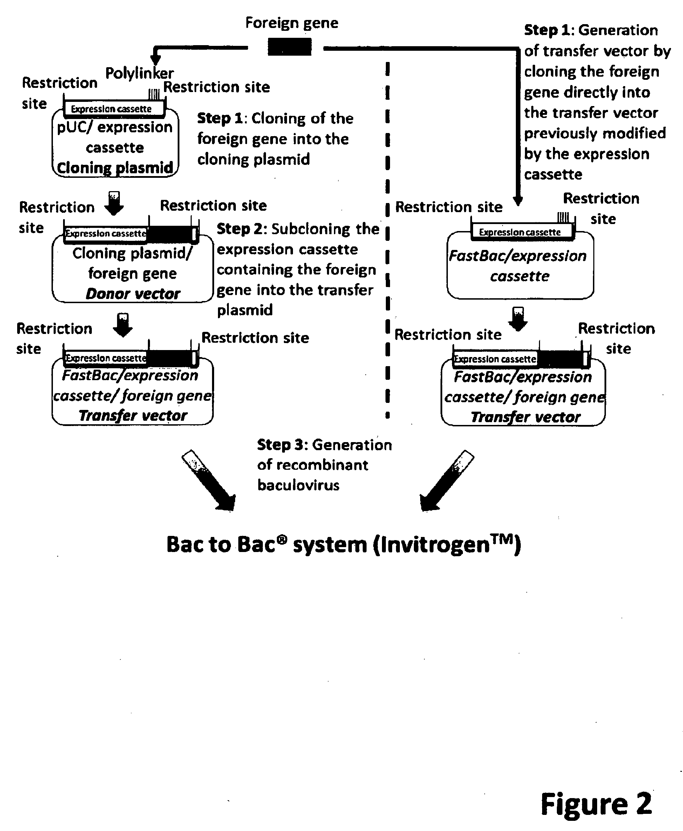

**Figure 2****:** Different strategies that result in the generation of recombinant baculoviruses by the "Bac-to-Bac^{®}" cloning system (invitrogen™).

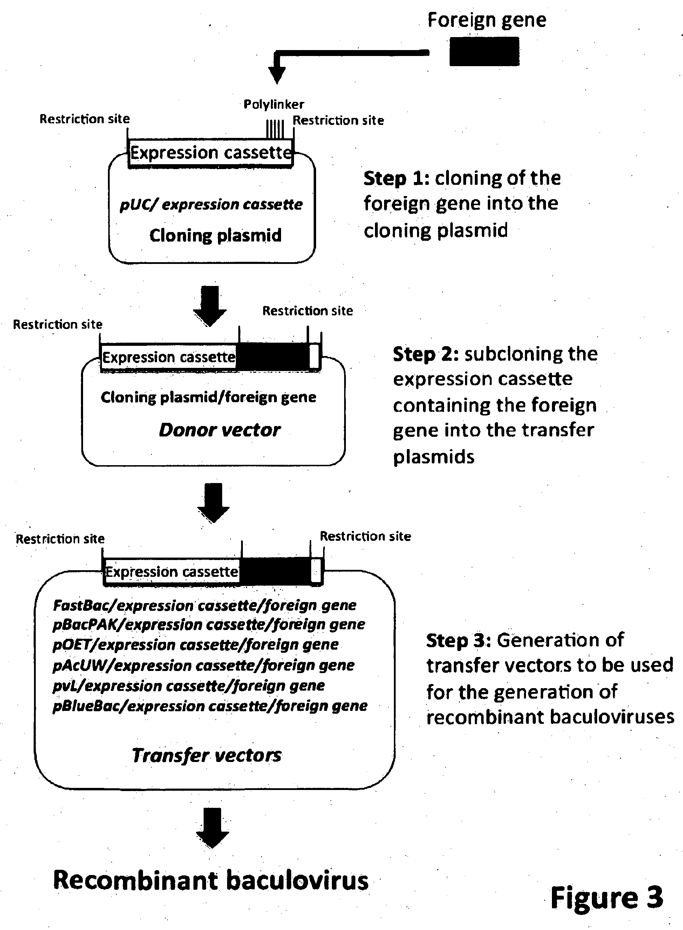

**Figure 3****:** General scheme for the generation of cloning, donor and transfer vectors compatible with other commercial technologies used to generate recombinant baculoviruses.

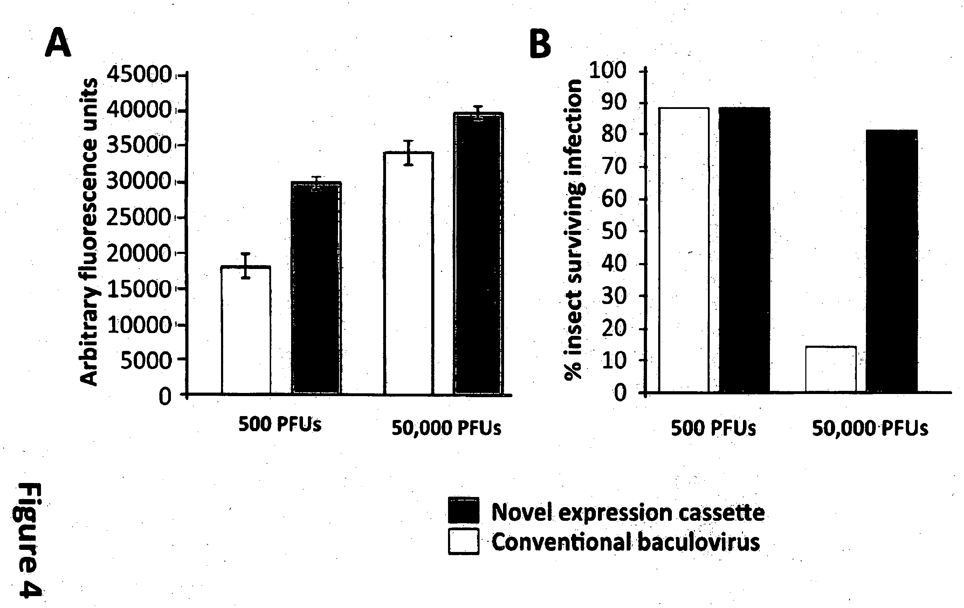

**Figure 4****: A)** Recombinant GFP production 96 hours post-infection in *Trichoplusia ni* insect larvae (fifth instar) inoculated with 500 or 50,000 PFUs of a conventional baculovirus expressing the protein under the control of the *polh* promoter or by a baculovirus containing the baculovirus cassette of the invention *polh Ac-ie-01*/*hr1p6.9p10GFP.* **B)** Percentage of larvae surviving 96 hours post-infection with the same baculoviruses as in panel A and at two different infectious doses, i.e. 500 and 50,000 PFUs.

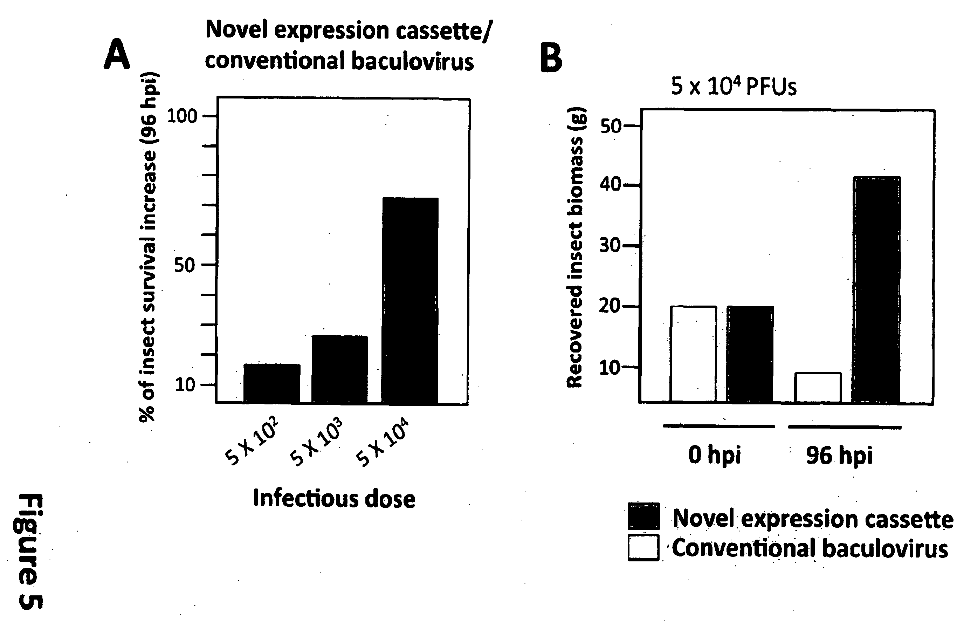

**Figure 5****: A)** Percentage of larvae surviving 96 hours post-infection with the baculovirus containing the expression cassette of the present invention *polhAc-ie-01*/*hr1p6.9p10GFP* with respect to a conventional baculovirus (*polhGFP*) at different infectious doses. **B)** Insect biomass at the moment of infection with a conventional baculovirus (*polhGFP*) or with a baculovirus containing the expression cassette of the present invention (*polhAc-ie-01*/*hr1p6.9p10GFP*) and the recovered biomass 96 hours post-infection. The infectious dose was 5 x 10⁴ PFUs.

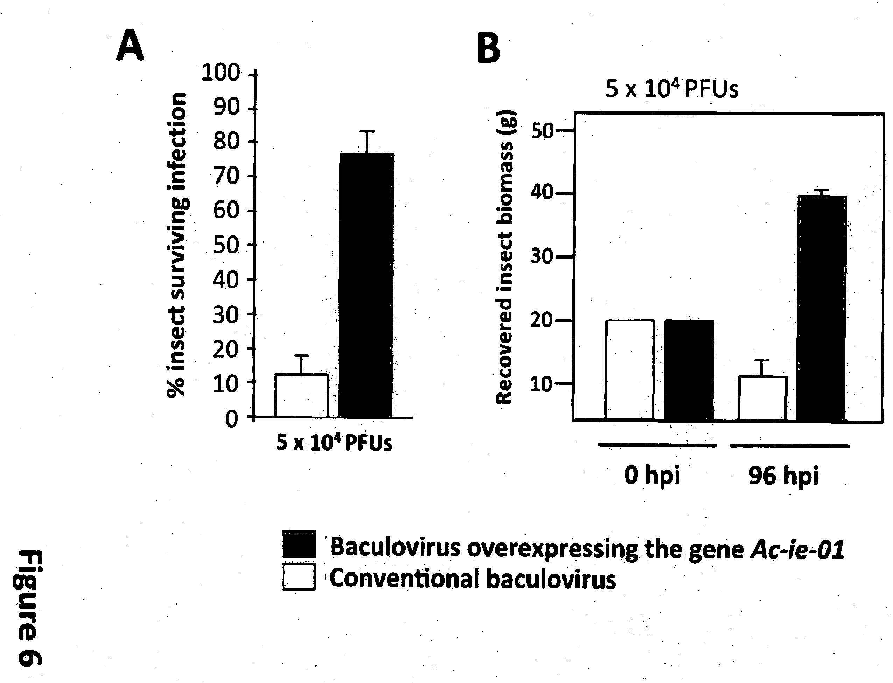

**Figure 6****: A)** Percentage of larvae surviving 96 hours post-infection using 5 x 10⁴ PFUs as the infectious dose of the baculovirus overexpressing the *Ac-ie-01* cDNA under the *polh* promoter (*polhAc-ie-01*) or using a conventional baculovirus expressing GFP under the *polh* promoter (*polhGFP*). **B)** Insect biomass at the time of infection with the same baculoviruses as in panel A and the recovered biomass at 96 hours post-infection. The infectious dose was 5 x 10⁴ PFUs.

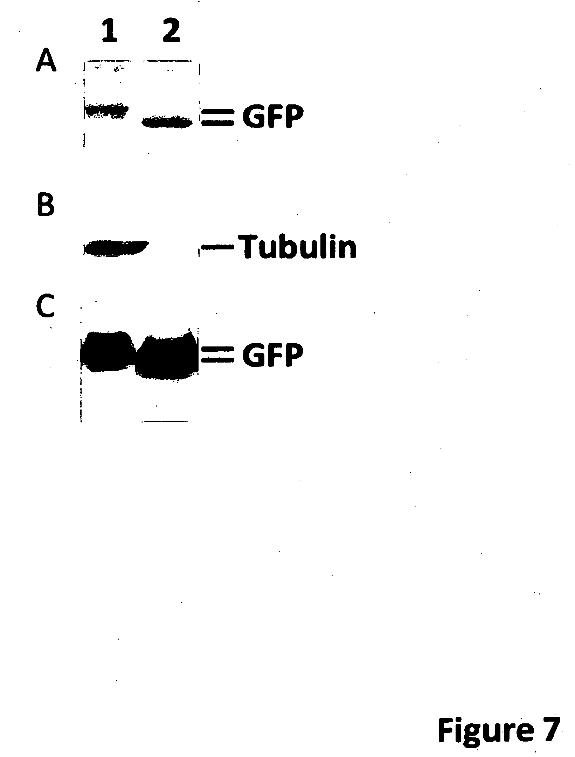

**Figure 7****:** Comparison of the recombinant GFP protein expressed by a conventional baculovirus under the control of the *polh* promoter (2) or by a baculovirus containing the expression cassette of the present invention *polhAc-ie-01*/*hr1p6.9p10GFP* (1). Extracts from larvae infected with 5 x 10⁵ PFUs of every baculovirus were obtained 96 hours post-infection. **A)** Coomassie blue staining of the recombinant GFP protein. **B)** Cell integrity after baculovirus infection determined by tubulin detection with a specific antiserum. C) Western blot detection of recombinant GFP protein by using a specific anti-GFP serum.

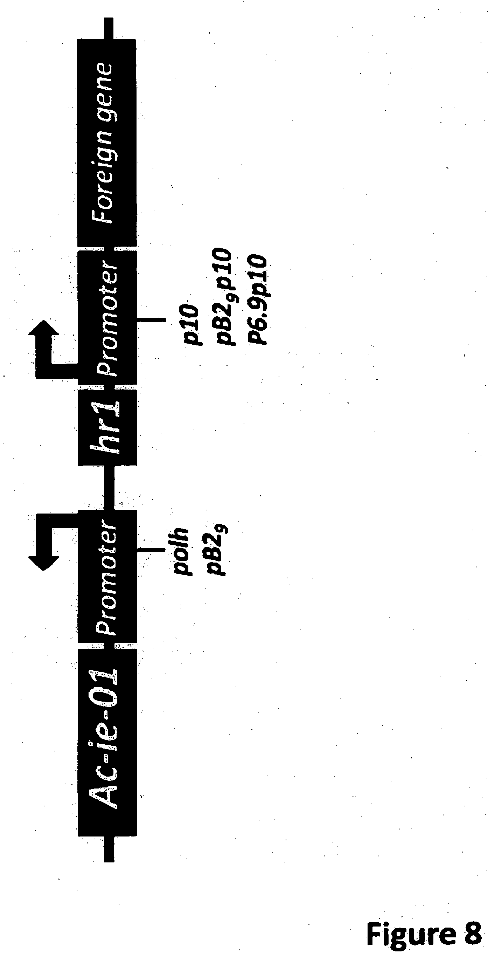

**Figure 8****:** Schematic representation of the preferred elements contained in the baculovirus expression cassettes of the invention, comprising encoding sequences for transcriptional regulators, homologous regions (*hr*) enhancing the transcription induced by promoter(s) of a foreign gene encoding a recombinant protein.

### DETAILED DESCRIPTION OF THE INVENTION

The present invention improves the expression of recombinant proteins by means of the introduction of recombinant DNA elements into insects.

These recombinant DNA elements of the present disclosure are sequences that cause the expression of baculovirus transcriptional regulators above endogenous levels and optionally enhancer homologous regions (*hr*) and promoters operably linked to these aforementioned elements.

Furthermore, the recombinant DNA elements may form part of an expression cassette.

"Expression cassette" refers to a nucleic acid sequence that contains recombinant DNA elements, which control (e.g. the promoter) and/or are required (e.g. the gene itself) for gene expression. The expression cassette can be introduced in a recombinant vector or baculovirus.

The recombinant DNA elements may be incorporated in a single nucleic acid sequence, cloning vector, transfer vector, recombinant baculovirus or cell. However, they can also be present in different nucleic acid sequences, cloning vectors, transfer vectors or recombinant baculoviruses and be introduced into the same cell.

The present invention surprisingly shows that introduction into insect larvae of sequences that cause the expression of baculovirus transcriptional regulators above endogenous levels obtained during baculovirus infection and the introduction of an enhancer homologous region (*hr*) sequence, a promoter or a combination of promoters is able to increase the production of a recombinant protein to unprecedented levels. This indicates the usefulness of this system for the expression of recombinant proteins *in vivo.*

Additionally, the introduction of these recombinant DNA elements into insect larvae with baculoviruses increases the survival rate of the larvae late after infection, especially after using high virus doses for infection (maximizes the recovered amount of biomass, i.e. the productivity of the system), as compared to larvae infected with a conventional baculovirus. The insect biomass recovered at the end of the production process is significantly increased as well.

Also, the integrity of the molecular cell machinery and cell morphology of said baculovirus-infected larvae is improved as compared to larvae infected with a conventional baculovirus. An improvement in the integrity of cell functions during baculovirus infection also contributes to the correct post-translational processing of the recombinant protein.

Thus, one aspect of the invention relates to an insect as defined the claims that contains a nucleic acid sequence that allows for the expression above endogenous levels of transcriptional regulators obtained during baculovirus infection. The transcriptional regulators are IE-1, IE-0 and/or fragments thereof.

"Transcriptional regulator" refers to a regulatory protein that has the ability to modulate the transcription of specific genes by, for example, binding to enhancer or repressor regions and/or recruiting further proteins that are involved in transcription.

IE-1 and its splice variant IE-0 are transcriptional regulators that are endogenously expressed during baculovirus infection. According to the present invention, IE-1, IE-0 and/or fragments thereof are recombinantly expressed to increase the total level of these proteins above endogenous levels. This is achieved through introducing further copies of the endogenous gene. Further copies of the endogenous genes can be introduced as transgenes under the control of a suitable promoter such as *polh* or *pB29.*

The expression level of the proteins IE-1, IE-0 and/or fragments thereof can be determined at both the mRNA and at the protein level with methods conventionally known to the person skilled in the art, such as quantitative PCR and Western Blot analysis.

According to the invention, IE-1, IE-0 and fragments thereof are encoded by the nucleic acids of SEQ ID NO: 1 (also referred to as *Ac-ie-01*) to SEQ ID NO: 5. SEQ ID NO: 1 is the *Ac-ie-01* cDNA that encodes both IE-1 and IE-0, SEQ ID NO: 2 is the coding sequence (CDS) of IE-1 and SEQ ID NO: 3 is the CDS of IE-0. SEQ ID NO: 4 and 5 are the CDSs of the N-terminal domains of IE-1 and IE-0 respectively that retain the catalytic transcriptional regulator activity. The proteins that are encoded by SEQ ID NO: 2-5 are represented by SEQ ID NO: 6-9 respectively.

The present disclosure furthermore discloses variants of SEQ ID NO: 1-9 that are or encode amino acids that are able to function as a transcriptional regulator. These variants are nucleic or amino acids whose nucleotide or amino acid sequence differs in one or more positions from these parental nucleic or amino acids, whereby differences might be additions, deletions and/or substitutions of nucleotides or amino acid residues.

Nucleic and amino acid sequences of the disclosure shall be distinguished from other nucleic and amino acid sequences by their degree of sequence identity or similarity respectively as determined using EMBOSS Needle with the default parameters (http://www.ebi.ac.uk/Tools/psa/emboss_needle/). Methods for the generation of such variants include random or site directed mutagenesis, site-saturation mutagenesis, PCR-based fragment assembly, DNA shuffling, homologous recombination *in vitro* or *in vivo,* and methods of gene-synthesis.

The sequence of the variants of SEQ ID NO: 1-5 is at least 70%, preferably at least 75%, more preferably at least 80%, more preferably at least 85%, more preferably at least 90% and most preferably at least 95% identical to the sequences of SEQ ID NO: 1-5.

The sequence of the variants of SEQ ID NO: 6-9 is at least 70%, preferably at least 75%, more preferably at least 80%, more preferably at least 85%, more preferably at least 90% and most preferably at least 95% similar to the sequences of SEQ ID NO: 6-9.

The insect is a lepidopter preferably selected from the group consisting of *Trichoplusia ni, Spodoptera frugiperda, Spodoptera exigua, Ascalapha odorata, Bombyx mori, Rachiplusia ni* and *Estigmene acrea.* In a preferred embodiment, the insect is a larva.

The insect of the present invention further contains, in addition to the nucleic acid sequence that allows for the expression above endogenous levels of the proteins IE-1, IE-0 and/or fragments thereof, a recombinant homologous region (*hr*) that can enhance the expression of a recombinant protein by being operably linked to the respective promoter.

Homologous regions, *hr,* are comprised of repeated units of about 70-bp with an imperfect 30-bp palindrome near their center. Homologous regions are repeated at eight locations in the *Ac*MNPV genome with 2 to 8 repeats at each side. Homologous regions have been implicated as both transcriptional enhancers and origins of baculovirus DNA replication.

"Enhancer region" refers to a control sequence, whose binding by transcriptional regulators increases the level of transcription of associated genes.

"Recombinant protein" refers to a protein that originates from recombinant DNA. Such proteins can be used for the benefit of humans and animals and may have industrial, commercial or therapeutic application.

"Being operably linked" refers to two nucleic acid sequences that are connected in a way that one influences the other in terms of, for example, transcriptional regulation.

"Promoter" refers to a DNA sequence to which RNA polymerase can bind to initiate transcription. The sequence may further contain binding sites for various proteins that regulate transcription, such as transcription factors. The promoter sequence may be composed of different promoter fragments (either different or the same fragments) that are localized closely in the DNA sequence and may be separated by linkers or spacer. Such promoters are referred to as chimeric promoters.

The enhancer homologous region sequence *hr* upstream of the promoter/s is preferably *hr1* (SEQ ID NO: 27). The promoters that drive the expression of the recombinant protein are selected from the group comprising SEQ ID NO: 10-16 or a sequence that is able to function as a promoter and has at least 70%, preferably at least 75%, more preferably at least 80%, more preferably at least 85%, more preferably at least 90% and most preferably at least 95% identity with the nucleotide sequence indicated in any of SEQ ID NO: 10-16.

In a preferred embodiment, the nucleic acid sequence comprises combinations of recombinant promoters, sequences encoding transcriptional regulators and enhancer regions selected from the group comprising SEQ ID NO: 17-26.

As indicated above, the recombinant promoters, sequences encoding transcriptional regulators and enhancer regions do not need to form part of a single molecule, instead these sequences may form part of distinct molecules as long as they are operably linked, i.e. contained within the same cells.

The insect of the present invention preferably comprises a nucleic acid sequence encoding a recombinant protein. This nucleic acid sequence is operably linked to the nucleic acid sequence that allows for the expression above endogenous levels of the proteins IE-1, IE-0 and/or fragments thereof and to a homologous region (*hr*).

The above described recombinant DNA elements are introduced into the insect by a recombinant baculovirus. Preferably, the baculovirus is *Ac*MNPV or *Bm*NPV and the insect an insect larva. The baculovirus is administered to the larva by oral administration *(per os)* or more preferably by injection. In a further embodiment the insect is infected, transfected, transduced or transformed with the recombinant baculovirus, transfer vector, cloning vector or nucleic acid sequence of the present invention. Preferably, the insect larvae are reared in a rearing module, such as described in the patent application ES 2 232 308.

In a further aspect, the invention discloses methods for producing a recombinant protein using the insect of the present invention. To this end, an insect can be infected, transfected, transduced or transformed with the recombinant baculovirus, transfer. vector, cloning vector or nucleic acid sequence. After expression of the recombinant protein, extraction and purification of the recombinant protein is done by conventional means.

In a preferred aspect for protein production, the larvae are infected by injecting a high virus dose (higher than 10⁴ Plaque Forming Units) of the recombinant bacutovirus 3-4 days after infection, the infected larvae are processed and the whole soluble protein extract is obtained by the use of appropriate extraction buffers. Extracts are centrifuged and the lipid fraction eliminated. Then, the recombinant protein is purified by conventional means.

The recombinant protein that is preferably produced by the methods of the present invention is a protein selected from the group comprising subunit monomeric vaccine, subunit multimeric vaccine, virus like particle, therapeutic protein, antibody, enzyme, cytokine, blood clotting factor, anticoagulant, receptor, hormone or diagnostic protein reagent

One aspect of the disclosure relates to the use of the recombinant baculovirus, transfer vector, cloning vector or nucleic acid sequence of the invention in a rearing, feeding or injection medium for an insect.

The present disdosure relates to a baculovirus that can be used to produce the insect of the present invention and comprises said sequence for expression above endogenous levels of the proteins IE-0, IE-1 and/or fragments thereof.

"Baculovirus" refers to a family of infectious viruses for invertebrates, mainly infecting insects and arthropods. A "recombinant baculovirus" has further introduced recombinant DNA through, for example, homologous recombination or transposition. The recombinant baculovirus preferably originates from *Ac*MNPV (*Autographa californica* nuclear polyhedrosis virus) or *Bm*NPV (*Bombyx mori* nucleopolyhedrovirus).

"Recombinant DNA" refers to a form of artificial DNA that is engineered through the combination or insertion of one or more DNA strands, thereby combining DNA that would normally not occur together.

"Recombinant DNA element" refers to a functional element within recombinant DNA, such as a promoter, enhancer or a gene. As mentioned above, the recombinant DNA elements of the present invention are sequences that cause the expression of baculovirus transcriptional regulators above endogenous levels, enhancer homologous regions (*hr*) and promoters operably linked to these aforementioned elements. Preferably, the baculovirus transcriptional regulators IE-1, IE-0 or fragments thereof are expressed above endogenous levels obtained during baculovirus infection.

The recombinant baculovirus contains in addition to (i) the sequence for expression above endogenous levels of the proteins IE-0, IE-1 and/or fragments thereof, (ii) a recombinant homologous region (*hr*) linked to (iii) a suitable promoter for driving the expression of a recombinant protein. The preferred combinations of these recombinant DNA elements are as described above for the insects. Furthermore, the recombinant baculovirus preferably contains a nucleic acid sequence encoding a recombinant protein.

The present disclosure further related to a transfer vector that can be used to produce the insec of the present invention and/or a recombihant baculovirus and comprises said sequence for expression above endogenous levels of the proteins IE-0, IE-1 and/or fragments thereof, in addition to a sequence suitable for Integration or transposition in a baculovirus.

Transfer vectors generally permit the insertion of genetic information into a baculovirus.

The transfer vector preferably contains in addition to (i) the sequence for expression above endogenous levels of the proteins IE-0, IE-1 and/or fragments thereof, (ii) a recombinant homologous region (hr) linked to (iii) a suitable promoter for driving the expression of a recombinant protein. The preferred combinations of these recombinant DNA elements are as described above for the insects.

In one preferred aspect, the transfer vector comprises a nucleic acid sequence encoding said recombinant protein, whereas in another preferred embodiment the transfer vector lacks such sequence.

In a preferred embodiment, the transfer vector is a bacmid.

"Bacmid" refers to a plasmid construct which contains the DNA sequence sufficient for generating a baculovirus when transfected into a cell.

In a further preferred aspect, the transfer vector is derived from any of the commercially available baculovirus expression systems "Bac-to-Bac^{®}" (invitrogen™), "BacPAK™" (Clontech™), "FlashBAC™" (Oxford Expression Technologies™), "BacuVance™" (GenScript™). "Bac-N-Blue DNA™" (invitrogen™), "BaculoDirect™" (invitrogen™), "BacVector®" 1000, 2000, 3000 (Novagen^{®}), "DiamondBac™" (Sigma-Aldrich^{®}) or "BaculoGold™" (BD biosciences™).

The present disclosure further relates to a cloning vector that can be used to produce the insect of the present invention a recombinant baculovirus and/or transfer vector and comprises said sequence for expression above endogenous levels of the proteins IE-0, IE-1 and/or fragments thereof, which is further suitable for bacterial replication.

"Cloning vector" refers to any vector that is suitable for cloning, which generally involves the presence of restriction sites, an origin of replication for bacterial propagation and a selectable marker.

The cloning vector preferably contains in addition to (i) the sequence for expression above endogenous levels of the proteins IE-0, IE-1 and/or fragments thereof, (ii) a recombinant homologous region (*hr*) linked to (iii) a suitable promoter for driving the expression of a recombinant protein. The preferred combinations of these recombinant DNA elements are as described above for the insects.

In one preferred aspect, the cloning vector comprises a nucleic acid sequence encoding said recombinant protein (also referred to as the "donor vector"), whereas in another preferred aspect the cloning vector lacks such sequence.

The present disclosure also relates to a nucleic acid sequence that can be used to produce the insect, recombinant baculovirus, transfer vector and/or cloning vector and comprises said sequence for expression above endogenous levels of the proteins IE-0, IE-1 and/or fragments thereof.

The nucleic acid sequence preferably contains in addition to (i) the sequence for expression above endogenous levels of the proteins IE-0, IE-1 and/or fragments thereof, (ii) a recombinant homologous region (*hr*) linked to (iii) a suitable promoter for driving the expression of a recombinant protein. The preferred combinations of these recombinant DNA elements are as described above for the insects.

In one preferred aspect, the nucleic acid sequence comprises a nucleic acid sequence encoding said recombinant protein, whereas in another preferred embodiment the nucleic acid sequence lacks such sequence.

**SUMMARY OF SEQUENCES**

| **SEQ ID NO:** | **Name:** |
|---|---|
| 1 | Complete *Ac-ie-01 c*DNA |
| 2 | Protein coding sequence (CDS) of IE-1 |
| 3 | CDS of IE-0 |
| 4 | CDS of the IE-1 N-terminal domain |
| 5 | CDS of the IE-0 N-terminal domain |
| 6 | IE-1 protein |
| 7 | IE-0 protein |
| 8 | IE-1 N-terminal domain protein |
| 9 | IE-0 N-terminal domain protein |
| 10 | *polh* (promoter) |
| 11 | *p10* (promoter) |
| 12 | *pB*29*p10* (promoter) |
| 13 | *p6.9p10* (promoter) |
| 14 | *pB29* (promoter) |
| 15 | *pB2p10* (promoter) |
| 16 | *polhp10* (promoter) |
| 17 | *polhAc-ie-01*/*hr1p10* |
| 18 | *polhAc-ie-01*/*hr1pB29p10* |
| 19 | *polhAc-ie-01*/*hr1p6.9p10* |
| 20 | *pB29Ac-ie-01lhr1p10* |
| 21 | *pB29Ac-ie-01*/*hr1pB29p10* |
| 22 | *pB29Ac-ie-01*/*hr1p6.9p10* |
| 23 | *polhAc-ie-01*/*hr1polh* |
| 24 | *pB29Ac-ie-01*/*hr1polh* |
| 25 | *polhAc-ie-01*/*hr1polhp10* |
| 26 | *pB29Ac-ie-01*/*hr1polhp10* |
| 27 | *Homologous region enhancer hr1* |
| 28 | *polhAc-ie-01* |
| 29 | *polhGFP* |

### DEPOSITION OF MICROORGANISMS ACCORDING TO THE BUDAPEST TREATY

Plasmids were deposited in the Spanish Type Culture Collection (CECT) (www.cect.org); University of Valencia, Parc Científic Universitat de València; Catedrático Agustín Escardino, 9; 46980 Paterna (Valencia), Spain, with the accession number CECT 8031, on the date October 4, 2011.

### EXAMPLES

### Example 1. Overexpression of baculovirus transcriptional regulators potentiates the enhancer function of a homologous region hr functionally linked to a promoter increasing recombinant protein expression in a baculovirus vector expression system (BEVS).

Immediate early viral proteins encoded by the *Ac-ie-01* cDNA, i.e. IE-1 and IE-0, from *Ac*MNPV are potent transcriptional regulators in baculoviruses. Transactivation mediated by these proteins is enhanced by their binding as a homodimer to the baculovirus homologous region (*hr*) sequences, which act as transcriptional enhancers. *Ac*MNPV IE-1/IE-0 are 67-72 kDa dimeric DNA-binding proteins that stimulate transcription in plasmid transfection assays through the activity of their N-terminal acidic domain (7, 8). Synthesized very early during infection, IE-1 and IE-0 accumulate within the nucleus, where they are maintained through late times. Using the dual plasmid pFastBac™ (invitrogen™), the *Ac-ie-01* cDNA was cloned under the control of the *polh* promoter. In the same plasmid, but in another locus, the GFP encoding gene was cloned downstream of the *hr1p6.9p10* chimeric promoter that was previously synthesized and contains the homologous region *hr1* fused to the promoters *p6.9* and *p10.* A schematic representation of the resulting baculovirus expression cassette of the present invention and the putative function of the recombinant DNA elements is shown in **Figure 1****.** The resulting plasmid was used to generate a recombinant baculovirus by the "Bac-to-Bac^{®}" system (invitrogen™). In parallel, a conventional baculovirus expressing the GFP protein under the control of *polh* promoter was generated by the same system.

The expression of GFP protein mediated by the different baculoviruses was studied by fluorimetry at 96 hours post-infection in *Trichoplusia ni* larvae using a low or high infectious dose (5 x 10² or 5 x 10⁴ respectively). The expression level of GFP was increased in larval extracts by the baculovirus containing above expression cassette by about 13 to more than 40% depending on the virus dose used **(****Figure 4A****).** Similar results were observed by using baculoviruses in which the *Ac-ie-01* cDNA was expressed under the control of the insect-derived *pB29* promoter or when the GFP was expressed under the control of the *hr1pB29p10* promoter (data not shown).

### Example 2. The baculovirus expression cassettes increase the baculovirus-infected insect larvae surviving rates and insect biomass recovered using high infectious doses through the transcriptional regulators encoded by the Ac-ie-01 cDNA

In the previous example an advantage of baculoviruses expressing the recombinant protein in the context of the baculovirus cassette in terms of protein productivity was shown. However, the main difference observed was in respect to the percentage of surviving larvae at high infectious doses (maximum productivity). Under such infection conditions, the baculovirus containing the expression cassette increased by about 70% the larvae survival rates **(****Figure 4B****).** This means that by using a conventional baculovirus the optimal infection conditions were using an infectious dose of 5 x 10² PFUs (maximum larvae surviving rate). In contrast, by using a baculovirus containing the expression cassette, a dose of 5 x 10⁴could be used, recovering the same number of larvae at the end of the production process **(****Figure 4B****).** Under such optimal production conditions (infection with 5 x 10⁴ PFUs), the baculovirus containing the expression cassette yields more than twice the amount of recombinant protein produced in insect larvae infected with the optimal dose for the conventional baculovirus (5 x 10² PFUs) **(****Figure 4** **A and B).**

Larvae infection studies using different infectious doses of a conventional baculovirus (*polhGFP*) or a baculovirus containing the expression cassette *polhAc-ie-01*/*hr1p6.9p10GFP,* both expressing the GFP proteins, revealed increasing surviving rates of infected larvae when the baculovirus containing the expression cassette was used (**Figure 5A****).** At the highest infectious dose (5 x 10⁴) the survival rate of larvae infected with the expression cassette increased by more than 70% as compared to larvae infected with a conventional baculovirus. This increase in infected larvae surviving rates had direct dramatic consequences in the insect biomass recovered at the end of the production process, with a 80% increase when the baculovirus containing the expression cassette was used at the highest infectious dose (maximum productivity) **(****Figure 5B****).**

To determine the genetic element/s responsible for such interesting properties related to the increase of survival rates after infection with high doses of the baculovirus, a recombinant baculovirus expressing the transcriptional regulators encoded by the *Ac-ie-01* cDNA under the control of the *polh* promoter was generated. Then, *T. ni* larvae were infected with a high infectious dose (5 x 10⁴ PFUs) of this baculovirus. As control, we used a baculovirus expressing the GFP protein under the control of the same promoter. Similarly to the larvae infected with the expression cassette *polhAc-ie-01*/*hr1p6.9p10GFP,* larvae infected with the baculovirus overexpressing the *Ac-ie-01* cDNA (*polhAc-ie-01*) also showed increased survival rates when compared with larvae infected with a conventional baculovirus expressing the GFP reporter protein under the control of the same promoter (*polhGPF*) **(****Figure 6A****).** This strongly suggests that the overexpression of the transcriptional regulators used in the baculovirus expression cassette protects the insect larvae from the baculovirus-induced mortality, allowing long-term expression (more recombinant protein production) and increasing the insect biomass recovery using high infectious doses (maximum productivity) (**Figure 6B****).**

### Example 3. Overexpression in a baculovirus expression system of transcriptional regulators encoded by the Ac-ie-01 cDNA facilitates the post-translational processing of recombinant proteins in infected insect larvae used as biofactories.

Cellular integrity during baculovirus infection is of great importance to ascertain the correct folding or any other post-transiational modification of foreign proteins expressed by this system. The baculovirus strong promoters commonly used for research and production, such as *polh* and *p10,* only express the foreign genes at late times post-infection when infected cells already show severe cytopathic effects and the cellular viability decreases. As described above, the overexpression of the transcriptional regulators used in the baculovirus expression cassette protects cells from pathogenic effects of the baculovirus infection, allowing a wide temporal window for recombinant protein production in cells remaining fully viable.

The relevance of the recombinant DNA elements incorporated into the expression cassette in relation to post-translational modifications of recombinant proteins in insect larvae as living biofactories was studied. For this purpose, a conventional baculovirus expressing the reporter protein GFP under the control of the *polh* promoter and a baculovirus incorporating the baculovirus cassette and also expressing the GFP protein (*polhAc-ie-01*/*hr1p6.9p10GFP*) were used to infect insect larvae at a dose of 5 x 10⁴ PFUs.

Infected larvae extracts were analysed at 96 hours post-infection by SDS-PAGE gels and Coomassie blue staining **(****Figure 7A****).** Interestingly, GFP protein expressed by a conventional baculovirus showed a band with a reduced molecular weight (lower than predicted), suggesting degradation or misfolding of the recombinant protein. In contrast, when the GFP protein expression was mediated by the baculovirus expression cassette the GFP band presented the expected molecular weight of this protein, i.e. about 27 kDa.

The infected larvae extracts were also analyzed by Western blot using anti-GFP monoclonal antibody (mab2515; Millipore™) (Figure 7C). The GFP protein was detected in larvae extracts infected by both baculoviruses, but showed a difference in electrophoretic mobility of the recombinant protein as observed by Coomassie blue staining.

In parallel, the integrity of the cell machinery was measured at different times post-infection by Western blot analysis of the cellular tubulin protein using a specific antiserum **(****Figure 7B****).** Infection with a conventional baculovirus impaired severely the cell integrity at 96 hours post-infection since the tubulin band detected decreased dramatically after this time point (degradation as a result of a complete loss of cell integrity). Consistent with the cellular protection induced by the recombinant DNA elements contained in the baculovirus expression cassette, cellular tubulin was not equally affected in cells infected by the recombinant baculovirus engineered with the expression cassette.

### Example 4. Cell culture and viruses.

The *Spodoptera frugiperda Sf21* or *Sf9* cell lines were cultured in 6-well tissue culture plates (1x10⁶ cells/well) in TNM-FH insect medium (Pan Biotech™, Germany) containing 10 % heat-inactivated fetal bovine serum (Pan Biotech™, Germany) at 27°C. *Ac*MNPV recombinant baculoviruses were obtained by the "Bac-to-Bac^{®}" Baculovirus Expression System (invitrogen™, USA). Different transfer vectors containing the recombinant DNA elements of the invention were generated using the pFastBac™-DUAL plasmid (invitrogen™). The promoters and regulatory elements incorporated into pFastBac™-DUAL were synthesized (GenScript™, USA) with the adequate flanking restriction sequences to facilitate the cloning. These transfer vectors were used to transfect *Sf*21 cells with Cellfectin® (invitrogen™, USA). The resulting recombinant baculoviruses from the infection of *Sf*21 cells were then passaged twice in cells and titered by the plaque assay method. The obtained gene constructs of the baculovirus expression cassettes are schematically shown in **Figure 8****,** showing different potential combinations of promoters driving the expression of the *Ac-ie-01* cDNA or the foreign gene (e.g. GFP). The different expression cassettes were used to generate the recombinant baculoviruses used in the examples shown in **Figures 4 to 7****.**

### Example 5. Generation of the cloning vector

The cloning vector is a small piece of DNA containing the baculovirus expression cassette into which a foreign DNA fragment can be inserted by treating the vehicle and the foreign DNA with a restriction enzyme that creates the same overhang, then ligating the fragments together. The essential characteristics of the cloning vector must include a synthetic multiple cloning site (MCS) to facilitate the insertion of foreign genes directed in a chosen orientation, a selectable marker, such as an antibiotic resistance to allow the selection of positively transformed cells and a functional origin of replication (ORI) for propagation in bacteria.

### Example 6. Generation of the donor vector containing the baculovirus expression cassette

A donor vector consists of a cloning vector, for example a pUC57 plasmid, containing the baculovirus expression cassette, into which a foreign gene has been cloned using the appropriate restriction enzymes. The baculovirus expression cassette was synthesized by ligating the following DNA sequences: (i) the baculovirus transcriptional regulator encoding sequence *Ac-ie-01* downstream of a promoter sequence, such as the *polh* or the *pB29* promoter, and upstream of the HSV TK polyadenylation signal and (ii) in another locus an enhancer sequence, for example, the homologous region *hr1,* upstream of (iii) a promoter sequence, for example, *pB29p10, p10, p6.9p10* or *polh,* followed by a multiple cloning site (MCS) for cloning the gene of interest and the SV40 polyadenylation signal downstream of the MCS **(****Figure 1****).** The baculovirus expression cassette is flanked by specific restriction sites (for example *Bgl*II and *BstZ17*l at the 5'-terminal end and *Bgl* II and *Sgf* I at the the 3'-terminal end) to facilitate subcloning into a transfer vector of a commercial baculovirus generation system (based on transposition, for example the "Bac-to-Bac^{®}" system (invitrogen™), or based. on homologous recombination, for example "flashBAC™" (Oxford Expression Technologies™), "Baculogold™" (BD Biosciences™), "BacPAK6™" (Clontech™), "Bac-N-Blue DNA™" (invitrogen™)) **(****Figure 2** **and** **3****).**

The encoding gene of the Green Fluorescence Protein (GFP) was cloned into the MCS of the cloning vector using the *Nco* I and *Spe* I restriction sites, generating the donor plasmid vector **(****Figure 2****).**

### Example 7. Generation of the transfer vector containing the baculovirus expression cassette.

The transfer vector was generated by digesting a donor vector with *BstZ17* I of the 5'-flanking site and with *Xba* I and cloning it into the transfer vector pFastBac™1 that was also digested with the same enzymes. In this case, as a result of the subcloning, the SV40 polyadenylation signal of the baculovirus expression cassette is exchanged by the SV40 polyadenlation signal from the transfer vector. Apart from this, all the elements of the expression cassette are included in the pFastBac transfer vector, substituting the *polh* promoter and MCS of the original commercial transfer vector **(****Figure 2****).**

### Example 8. Generation of the baculovirus expression vector containing the baculovirus expression cassette using the "Bac-to-Bac^{®}" system

The modified transfer vector pFastBac™1 and the individual baculovirus expression cassette were used to generate a recombinant baculovirus by using the "Bac-to-Bac^{®}" Baculovirus Expression System. More specifically, the modified transfer vector was used to transform the *E. coli* host strain DH10Bac™ that contains a baculovirus shuttle vector (bacmid) and a helper plasmid, and allows the generation of a recombinant bacmid following transposition of the expression cassette. The DNA of the recombinant bacmid containing the baculovirus expression cassette and the GFP encoding gene was then used to transfect insect cells, for example, *Sf*21 cells, using Cellfectin^{®}. 72 hours post-transfection, cells were harvested and the first recombinant baculovirus generation was obtained (Figure 2). This recombinant baculovirus could then be further amplified and/or titered following conventional protocols. Similar procedures can be used to generate recombinant baculoviruses with other transfer vectors provided by commercial BEVSs **(****Figure 3**).

### Example 9. Rearing and infection of insect larvae.

*Trichoplusia ni* (cabbage looper) larvae were reared under level 2 biosafety conditions. Eggs were placed into specially designed larva developmental cages containing an artificial insect diet and were kept in growth chambers at 22 °C under controlled humidity (50%) and light period (8 h/day) conditions.

Fifth-instar larvae (last instar larvae before pupation), were used for all infection experiments. The standard weight of each larva was approximately 120-130 mg and larvae were injected near the proleg (forward of the body cavity) with 5 µl of recombinant baculoviruses diluted to reach the number of plaque forming units (PFU) per dose selected. Larvae were processed at 96 hpi. The larvae collected were frozen immediately to be stored at -20°C until they were processed for recombinant protein quantification. Total soluble, non-denatured proteins (TSNDPs) from frozen *T. ni* larvae infected by the baculoviruses were obtained by homogenization using a Bag Mixer^{®} blender (Interscience™, France) for 2 min. Extraction buffer was composed of PBS 1x, Triton X-100 at 0.01%, Complete protease inhibitor cocktail (Roche™, Germany), and DTT 25 mM.

### Example 10. Fluorimetric analysis.

About 20 µg of total soluble proteins derived from infected cells, containing different amounts of recombinant GFP protein, were analyzed and quantified by a Tecan™ GENios™ (CA, USA) fluorescence plate reader (excitation [Ex], 485/emission [Em], 535).

### Example 11. Western blot analysis.

Total soluble protein fractions (10 µg) from larvae infected with the recombinant baculoviruses were resolved in 15% SDS-PAGE gels. Gels were stained by the Coomassie blue staining method or transferred to nitrocellulose membranes. Western blots were probed with the anti-GFP monoclonal antibody mab2515 (Millipore™, USA) or tubulin antiserum (T5168; Sigma-Aldrich™) at 1:1000 and the immunocomplexes were revealed with anti-mouse IgG-horseradish peroxidase (HRP)-labeled conjugate (KPL™, UK), diluted 1:2,000 as a secondary antibody. Protein bands were detected using an ECL western blotting detection system and analyzed by the ChemiDoc™ XRS Gel Imaging System (Bio-Rad™, USA).

### BIBLIOGRAPHY

1. Nettleship, J.E., Assenberg, R., Diprose, J.M., Rahman-Huq, N., Owens, R.J. Recent advances in the production of proteins in insect and mammalian cells for structural biology. J. Struct Biol. 2010, 172, 55-65.
2. Gomez-Casado E, Gomez-Sebastian S, Núñez MC, Lasa-Covarrubias R, Martínez-Pulgarín S, Escribano JM. Insect larvae biofactories as a platform for influenza vaccine production. Protein Expr Purif 79: 35-43. 2011.
3. Smith, G.E., M.D. Summers, and M.J. Fraser. 1983. Production of human beta interferon in insect cells infected with a baculovirus expression vector. Mol. Cell. Biol. 3: 2156-2165.
4. Tomita, M., Munetsuna, H., Sato, T., Adachi, T., Hino, R., Hayashi, M., Shimizu, K., Nakamura, N., Tamura, T., Yoshizato, K., 2003. Transgenic silkworms produce recombinant human type III procollagen in cocoons. Nat. Biotechnol. 21 (1), 52-56.
5. Perez-Filgueira, D.M, Resino-Talavan, P., Cubillos, C., Angulo, I., Barderas, M.G., Barcena, J., Escribano, J.M., 2007. Development of a low-cost, insect larvaederived recombinant subunit vaccine against RHDV. Virology 364 (2), 422-430.
6. Perez-Martin, E., Gomez-Sebastian, S., Argilaguet, J.M., Sibila, M., Fort, M., Nofrarias, M., Kurtz, S., Escribano, J.M., Segales, J., Rodriguez, F., 2010. Immunity conferred by an experimental vaccine based on the recombinant PCV2 Cap protein expressed in Trichoplusia ni-larvae. Vaccine 28 (11), 2340-2349.
7. Fernández-San Millán A, Gómez-Sebastián S, Nunez MC, Veramendi J, Escribano JM. Human papillomavirus-like particles vaccine efficiently produced in a non-fermentative system based on insect larva. Protein Expr. Purif. 2010, 74: 1-8
8. Gomez-Casado E, Gomez-Sebastian S, Núñez MC, Lasa-Covarrubias R, Martínez-Pulgarín S, Escribano JM. Insect larvae biofactories as a platform for influenza vaccine production Protein Expr. Purif. 2011, 79: 35-43
9. J.A. Medin, L. Hunt, K. Gathy, R.K. Evans, M.S. Coleman, Efficient, low-cost protein factories: expression of human adenosine deaminase in baculovirusinfected insect larvae, Proc. Natl Acad. Sci. USA 87 (1990) 2760-2764.
10. N.M. Tremblay, B.P. Kennedy, I.P. Street, W.J. Kaupp, F. Laliberte, P.K. Weech, Human group II phospholipase A2 expressed in Trichoplusia ni larvae -isolation and kinetic properties of the enzyme, Protein Expr. Purif. 4 (1993) 490-498.
11. U. Reis, B. Blum, B.U. von Specht, H. Domdey, J. Collins, Antibody production in silkworm cells and silkworm larvae infected with a dual recombinant Bombyx mori nuclear polyhedrosis virus, Biotechnology (NY) 10 (1992) 910-912.
12. F. Gil, M. Perez-Filgueira, M.G. -Barderas, C. Pastor-Vargas, C. Alonso, F. Vivanco, J.M. Escribano, Targeting antigens to an invariant epitope of the MHC Class II DR molecule potentiates the immune response to subunit vaccines, Virus Res. (2010).
13. S. Mathavan, V.T. Gautvik, E. Rokkones, O.K. Olstad, B.N: Kareem, S. Maeda, K.M. Gautvik, High-level production of human parathyroid hormone in Bombyx mori larvae and BmN cells using recombinant baculovirus, Gene 167 (1995) 33-39.
14. S. Sumathy, V.B. Palhan, K.P. Gopinathan, Expression of human growth hormone in silkworm larvae through recombinant Bombyx mori nuclear polyhedrosis virus, Protein Expr. Purif. 7 (1996) 262-268.
15. X. Shi, J. Qin, J. Zhu, D. Zhu, Expression of biologically active human granulocyte-macrophage colony-stimulating factor in the silkworm (Bombyx mori), Biotechnol. Appl. Biochem. 24 (Pt 3) (1996) 245-249.
16. M.Q. Pham, S. Naggie, M. Wier, H.J. Cha, W.E. Bentley, Human interleukin-2 production in insect (Trichoplusia ni) larvae: effects and partial control of proteolysis, Biotechnol. Bioeng. 62 (1999) 175-182.
17.J.B. Katz, A.L. Shafer, K.A. Eernisse, Construction and insect larval expression of recombinant vesicular stomatitis nucleocapsid protein and its use in competitive ELISA, J. Virol. Methods 54 (1995) 145-157.
18. D.M. Perez-Filgueira, F. Gonzalez-Camacho, C. Gallardo, P. Resino-Talavan, E. Blanco, E. Gomez-Casado, C. Alonso, J.M. Escribano, Optimization and validation of recombinant serological tests for African swine fever diagnosis based on detection of the p30 protein produced in Trichoplusia ni larvae, J. Clin. Microbiol. 44 (2006) 3114-3121.
19. S. Gomez-Sebastian, D.M. Perez-Filgueira, E. Gomez-Casado, M.C. Nunez, I. Sanchez-Ramos, E. Tabares, J.M. Escribano, DIVA diagnostic of Aujeszky's disease using an insect-derived virus glycoprotein E, J. Virol. Methods 153 (2008) 29-35.
20. F. Todoli, M. Perez-Filgueira, I. Galindo, S. Gomez-Sebastian, J.M. Escribano, A. Rodriguez-Cortes, J. Alberola, Seroreactivity against raw insect-derived recombinant KMPII, TRYP, and LACK Leishmania infantum proteins in infected dogs, Vet. Parasitol. 164 (2009) 154-161.
21. Hill-Perkins MS, Possee RD. A baculovirus expression vector derived from the basic protein promoter of Autographa californica nuclear polyhedrosis virus. J Gen Virol. 1990, 71 (Pt 4):971-6.
22. Passarelli, A. L., and L. K. Miller. Three baculovirus genes involved in late and very late gene expression: ie-1, ie-n, and lef-2. J. Virol. 1993, 67:2149-2158
23. Rodems, S. M., S. S. Pullen, and P. D. Friesen. DNA-dependent transregulation by IE1 of Autographa californica nuclear polyhedrosis virus: IE1 domains required for transactivation and DNA binding. J. Virol. 1997, 71: 9270-9277.
24. Lin X, Chen Y, Yi Y, Zhang Z: Baculovirus immediately early 1, a mediator for homologous regions enhancer function in trans. Virol J 2010, 7:32.
25. Okano K, Mikhailov VS, Maeda S: Colocalization of baculovirus IE-1 and two DNA-binding proteins, DBP and LEF-3, to viral replication factories. Journal of virology 1999, 73(1):110-119.

## Claims

1. Insect derived from the genus Lepidoptera, comprising nucleic acid elements introduced into the insect by a recombinant baculovirus containing an endogenous *Ac-ie-01* gene, wherein said nucleic acid elements comprise
[1] a further copy of baculoviral *Ac-ie-01* cDNA as transgene under the control of a suitable promoter that allows for the expression of the proteins IE-1, IE-0 and/or fragments thereof functioning as transcriptional regulators above endogenous levels obtained during baculovirus infection, wherein the nucleic acid is selected from the group consisting of:
(a) a nucleic acid containing the nucleotide sequence indicated in any of SEQ ID NO: 1-5;
(b) a nucleic acid sequence having a sequence identity of at least 70%, preferably at least 75%, more preferably at least 80%, more preferably at least 85%, more preferably at least 90% and most preferably at least 95% with the nucleotide sequence indicated in any of SEQ ID NO: 1-5 and encoding a protein able to function as a transcriptional regulator in a recombinant baculovirus;
(c) a nucleic acid sequence encoding an amino acid containing the amino acid sequence indicated in any of SEQ ID NO: 6-9; and
(d) a nucleic acid sequence encoding an amino acid sequence having a sequence similarity of at least 70%, preferably at least 75%, more preferably at least 80%, more preferably at least 85%, more preferably at least 90% and most preferably at least 95% with the amino acid sequence indicated in any of SEQ ID NO: 6-9 and able to function as a transcriptional regulator in a recombinant baculovirus;
[2] wherein the insect further comprises at least one recombinant homologous region (hr) as enhancer region, operably linked to any promoter that is suitable for driving the expression of a recombinant protein,
[3] wherein the promoter that drives the expression of said recombinant protein is selected from the group of nucleic acids comprising:
i. a nucleic acid containing the nucleotide sequence indicated in any of SEQ ID NO: 10-16; and
ii. a nucleic acid sequence able to function as a promoter in a recombinant baculovirus and having a sequence identity of at least 70%, preferably at least 75%, more preferably at least 80%, more preferably at least 85%, more preferably at least 90% and most preferably at least 95% with the nucleotide sequence indicated in any of SEQ ID NO: 10-16.

2. Insect according to claim 1, wherein the recombinant homologous region (hr) is the sequence indicated in SEQ ID NO: 27 (*hr1*).

3. Insect according to any of the claims 1-2, wherein the nucleic acid sequence that comprises combinations of recombinant promoters, sequences encoding transcriptional regulators and enhancer regions are selected from the group comprising SEQ ID NO: 17-26.

4. Insect according to any of the claims 1-43, wherein the insect is selected from the group consisting of *Trichoplusia ni*, *Spodoptera frugiperda, Spodoptera exigua, Ascalapha odorata*, *Bombyx mori, Rachiplusia ni* and *Estigmene* acrea.

5. Insect according to any of the claims 1-4, wherein the nucleic acid sequence(s) is introduced into the insect by a recombinant baculovirus selected from *Ac*MNPV or *Bm*NPV.

6. Insect according to any of the claims 1-5, further comprising a nucleic acid sequence encoding a recombinant protein, wherein said nucleic acid sequence is operably linked to a nucleic acid sequence selected from the group consisting of the nucleic acid sequences of claims 1-3.

7. Method for producing a recombinant protein that comprises the use of an insect of claim 6 and the extraction and purification of the recombinant protein by conventional means.

8. Method for producing a recombinant protein according to claim 7, wherein the recombinant protein is selected from the group comprising subunit monomeric vaccine, subunit multimeric vaccine, virus like particle, therapeutic protein, antibody, enzyme, cytokine, blood clotting factor, anticoagulant, receptor, hormone or diagnostic protein reagent.

## Patentansprüche

1. Insekt, abgeleitet von der Gattung Lepidoptera, umfassend Nukleinsäureelemente, die durch einen rekombinanten Baculovirus, welcher ein endogenes *Ac-ie-01*-Gen enthält, in das Insekt eingebracht wurden, wobei die Nukleinsäureelemente umfassen
(1) eine weitere Kopie von Baculovirus-*Ac-ie-01*-cDNA als Transgen unter der Kontrolle eines geeigneten Promotors, welcher die Expression der Proteine IE-1, IE-0 und/oder von Fragmenten davon, die als Transkriptionsregulatoren funktionieren, oberhalb endogener Niveaus erlaubt, erhalten während der Baculovirus-Infektion, wobei die Nukleinsäure ausgewählt ist aus der Gruppe, bestehend aus:
(a) einer Nukleinsäure, welche die in irgendeiner der SEQ ID NO: 1-5 angegebene Nukleotidsequenz enthält;
(b) einer Nukleinsäuresequenz, welche eine Sequenzidentität von wenigstens 70%, bevorzugt wenigstens 75%, bevorzugter wenigstens 80%, bevorzugter wenigstens 85%, bevorzugter wenigstens 90% und am meisten bevorzugt wenigstens 95% zu der in irgendeiner der SEQ ID NO: 1-5 angegebenen Nukleotidsequenz hat und ein Protein codiert, welches dazu fähig ist, in einem rekombinanten Baculovirus als Transkriptionsregulator zu funktionieren;
(c) einer Nukleinsäuresequenz, welche eine Aminosäure codiert, die die in irgendeiner der SEQ ID NO: 6-9 angegebene Aminosäuresequenz enthält; und
(d) einer Nukleinsäuresequenz, welche eine Aminosäuresequenz codiert, die eine Sequenzähnlichkeit von wenigstens 70%, bevorzugt wenigstens 75%, bevorzugter wenigstens 80%, bevorzugter wenigstens 85%, bevorzugter wenigstens 90% und am meisten bevorzugt wenigstens 95% zu der in irgendeiner der SEQ ID NO: 6-9 angegebenen Aminosäuresequenz hat und dazu fähig ist, in einem rekombinanten Baculovirus als Transkriptionsregulator zu funktionieren;
(2) wobei das Insekt weiterhin wenigstens eine rekombinante homologe Region (hr) als Enhancer-Region umfasst, die funktionsfähig mit einem Promotor verknüpft ist, welcher zum Steuern der Expression eines rekombinanten Proteins geeignet ist,
(3) wobei der Promotor, welcher die Expression des rekombinanten Proteins steuert, aus der Gruppe von Nukleinsäuren ausgewählt ist, welche umfasst:
i. eine Nukleinsäure, welche die in irgendeiner der SEQ ID NO: 10-16 angegebene Nukleotidsequenz enthält; und
ii. eine Nukleinsäuresequenz, die dazu fähig ist, in einem rekombinanten Baculovirus als Promotor zu funktionieren, und eine Sequenzidentität von wenigstens 70%, bevorzugt wenigstens 75%, bevorzugter wenigstens 80%, bevorzugter wenigstens 85%, bevorzugter wenigstens 90% und am meisten bevorzugt wenigstens 95% zu der in irgendeiner der SEQ ID NO: 10-16 angegebenen Nukleotidsequenz hat.

2. Insekt gemäß Anspruch 1, wobei die rekombinante homologe Region (*hr*) die in SEQ ID NO: 27 angegebene Sequenz ist (*hr1*).

3. Insekt gemäß irgendeinem der Ansprüche 1-2, wobei die Nukleinsäuresequenz, welche Kombinationen von rekombinanten Promotoren, Transkriptionsregulatoren codierenden Sequenzen und Enhancer-Regionen umfasst, aus der Gruppe ausgewählt ist, welche aus SEQ ID NO: 17-26 umfasst.

4. Insekt gemäß irgendeinem der Ansprüche 1-3, wobei das Insekt aus der Gruppe ausgewählt ist, bestehend aus *Trichoplusia ni, Spodoptera frugiperda, Spodoptera exigua, Ascalapha odorata, Bombyx mori, Rachiplusia ni* und *Estigmene acrea.*

5. Insekt gemäß irgendeinem der Ansprüche 1-4, wobei die Nukleinsäuresequenz(en) durch einen rekombinanten Baculovirus, ausgewählt unter *Ac*MNPV oder *Bm*NPV, in das Insekt eingebracht wird/werden.

6. Insekt gemäß irgendeinem der Ansprüche 1-5, welches weiterhin eine Nukleinsäuresequenz umfasst, welche ein rekombinantes Protein codiert, wobei die Nukleinsäuresequenz funktionsfähig mit einer Nukleinsäuresequenz verknüpft ist, ausgewählt aus der Gruppe, bestehend aus den Nukleinsäuresequenzen der Ansprüche 1-3.

7. Verfahren zum Produzieren eines rekombinanten Proteins, welches die Verwendung eines Insekts gemäß Anspruch 6 und die Extraktion und Reinigung des rekombinanten Proteins durch konventionelle Mittel umfasst.

8. Verfahren zum Produzieren eines rekombinanten Proteins gemäß Anspruch 7, wobei das rekombinante Protein aus der Gruppe ausgewählt ist, welche umfasst: monomeren Untereinheiten-Impfstoff, multimeren Untereinheiten-Impfstoff, Virus-artiges Partikel, therapeutisches Protein, Antikörper, Enzym, Zytokin, Blutgerinnungsfaktor, Antikoagulans, Rezeptor, Hormon oder diagnostisches Proteinreagens.

## Revendications

1. Insecte dérivé du genre Lepidoptera, comprenant des éléments d'acide nucléique introduits dans l'insecte par un baculovirus recombinant contenant un gène *Ac-ie-01* endogène, dans lequel lesdits éléments d'acide nucléique comprennent
[1] une autre copie de l'ADNc *Ac-ie-01* baculoviral en tant que transgène sous le contrôle d'un promoteur convenable qui permet l'expression des protéines IE-1, IE-0 et/ou de fragments de celles-ci jouant le rôle de régulateurs transcriptionnels au-dessus de niveaux endogènes obtenus pendant une infection par un baculovirus, dans lequel l'acide nucléique est choisi dans le groupe constitué de :
(a) un acide nucléique contenant la séquence nucléotidique indiquée dans l'une quelconque de SEQ ID N° : 1 à 5 ;
(b) une séquence d'acide nucléique ayant une identité de séquence d'au moins 70 %, de préférence d'au moins 75 %, de manière davantage préférée d'au moins 80 %, de manière davantage préférée d'au moins 85 %, de manière davantage préférée d'au moins 90% et de la manière la plus préférée d'au moins 95% avec la séquence nucléotidique indiquée dans l'une quelconque de SEQ ID N° : 1 à 5, et codant une protéine capable de jouer le rôle de régulateur transcriptionnel dans un baculovirus recombinant;
(c) une séquence d'acide nucléique codant un acide aminé contenant la séquence d'acides aminés indiquée dans l'une quelconque de SEQ ID N° : 6 à 9 ; et
(d) une séquence d'acide nucléique codant une séquence d'acides aminés ayant une similitude de séquence d'au moins 70%, de préférence d'au moins 75%, de manière davantage préférée d'au moins 80 %, de manière davantage préférée d'au moins 85%, de manière davantage préférée d'au moins 90% et de la manière la plus préférée d'au moins 95 % avec la séquence d'acides aminés indiquée dans l'une quelconque de SEQ ID N° : 6 à 9, et capable de jouer le rôle de régulateur transcriptionnel dans un baculovirus recombinant;
[2] dans lequel l'insecte comprend en outre au moins une région homologue recombinante (*hr*) en tant que région d'activateur, liée de façon opérationnelle à un quelconque promoteur qui convient à l'entraînement de l'expression d'une protéine recombinante,
[3] dans lequel le promoteur qui entraîne l'expression de ladite protéine recombinante est choisi dans le groupe d'acide nucléique comprenant :
i. un acide nucléique contenant la séquence nucléotidique indiquée dans l'une quelconque de SEQ ID N° : 10 à 16 ; et
ii. une séquence d'acide nucléique capable de jouer le rôle de promoteur dans un baculovirus recombinant et ayant une identité de séquence d'au moins 70%, de préférence d'au moins 75 %, de manière davantage préférée d'au moins 80 %, de manière davantage préférée d'au moins 85 %, de manière davantage préférée d'au moins 90% et de la manière la plus préférée d'au moins 95% avec la séquence nucléotidique indiquée dans l'une quelconque de SEQ ID N° : 10 à 16.

2. Insecte selon la revendication 1, dans lequel la région homologue recombinante (*hr*) est la séquence indiquée dans SEQ ID N° : 27 (*hr1*).

3. Insecte selon l'une quelconque des revendications 1 et 2, dans lequel la séquence d'acide nucléique qui comprend des combinaisons de promoteurs recombinants, des séquences codant des régulateurs transcriptionnels et des régions d'activateur est choisie dans le groupe comprenant SEQ ID N° : 17 à 26.

4. Insecte selon l'une quelconque des revendications 1 à 3, dans lequel l'insecte est choisi dans le groupe constitué de *Trichoplusia ni, Spodoptera frugiperda, Spodoptera exigua, Ascalapha odorata, Bombyx mori, Rachiplusia ni* et *Estigmene acrea.*

5. Insecte selon l'une quelconque des revendications 1 à 4, dans lequel la ou les séquences d'acide nucléique sont introduites dans l'insecte par un baculovirus recombinant choisi parmi *Ac*MNPV ou *Bm*NPV.

6. Insecte selon l'une quelconque des revendications 1 à 5, comprenant en outre une séquence d'acide nucléique codant une protéine recombinante, dans lequel ladite séquence d'acide nucléique est liée de façon opérationnelle à une séquence d'acide nucléique choisie dans le groupe constitué des séquences d'acide nucléique des revendications 1 à 3.

7. Procédé de production d'une protéine recombinante qui comprend l'utilisation d'un insecte selon la revendication 6, et l'extraction et la purification de la protéine recombinante par des moyens classiques.

8. Procédé de production d'une protéine recombinante selon la revendication 7, dans lequel la protéine recombinante est choisie dans le groupe comprenant un vaccin monomère sous-unitaire, un vaccin multimère sous-unitaire, une particule de type virus, une protéine thérapeutique, un anticorps, une enzyme, une cytokine, un facteur de coagulation sanguine, un anticoagulant, un récepteur, une hormone ou un réactif de diagnostic de type protéine.


### SEQUENCE LISTING

<110> ALTERNATIVE GENE EXPRESSION S.L.
<120> RECOMBINANT DNA ELEMENTS FOR THE EXPRESSION OF RECOMBINANT PROTEINS IN INSECTS
<130> 156 594
<150> EP11169634.0
   <151> 2011-06-10
<150> PCT/EP2011/068381
   <151> 2011-10-20
<150> PCT/EP2011/072406
   <151> 2011-12-12
<160> 29
<170> PatentIn version 3.5
<210> 1
   <211> 1911
   <212> DNA
   <213> Autographa californica nucleopolyhedrovirus
<400> 1
   atgatccgta catccagcca cgtcctgaac gtccaagaaa acatcatgac ttccaactgt      60
   gcttccagcc cctactcctg tgaggccact tcagcctgcg ctgaggccca gcaactgcag     120
   gtggacacag gtggcgataa gatcgtgaac aaccaggtca ccatgactca aatcaacttc     180
   aacgcttcct acacctctgc cagcactccc tctcgtgcta gcttcgacaa ctcatactcg     240
   gagttctgcg acaagcaacc taacgattac ttgtcttact acaaccaccc aaccccggac     300
   ggagctgata ctgtcatctc cgactctgaa accgctgccg ctagcaactt cctcgcctca     360
   gttaactcgc tcactgacaa cgatttggtg gagtgtctgc tcaagaccac tgacaacctg     420
   gaggaagctg tgtcctctgc ctactacagc gagtcactcg aacagccagt ggtcgaacaa     480
   ccctctccta gctcagctta ccacgccgag tccttcgaac actctgctgg tgtcaaccag     540
   ccgtcggcca caggcaccaa gaggaagttg gacgagtacc tggataactc ccagggagtt     600
   gtgggtcaat tcaacaagat caagttgaga cctaagtaca agaagagcac catccagtca     660
   tgcgctacac tggaacaaac catcaaccac aacactaaca tctgtacagt ggcttccacc     720
   caggagatca ctcactactt cacaaacgac ttcgccccct acctgatgag gttcgacgat     780
   aacgactaca actcgaacag attctccgat cacatgtctg aaaccggtta ctacatgttc     840
   gtcgttaaga agtccgaggt gaagcctttc gaaatcatct tcgccaagta cgtctctaac     900
   gtggtctacg agtacacaaa caactactac atggttgaca accgtgtgtt cgttgtgacc     960
   ttcgataaga tccgcttcat gatcagctac aacctggtta aggagactgg catcgaaatc    1020
   ccacactcac aggacgtctg caacgatgag accgccgctc aaaactgcaa gaagtgtcac    1080
   ttcgtggacg tccaccacac attcaaggcc gctctgacct cctacttcaa cctcgatatg 1140
   tactacgctc agacaacctt cgtgaccttg ctgcaatcac tcggcgagcg taagtgtgga 1200
   ttcctcttgt cgaagttgta cgagatgtac caggacaaga acctcttcac tttgcccatc 1260
   atgctgagcc gcaaggaatc aaacgagatc gaaaccgcct ctaacaactt cttcgtctcg 1320
   ccatacgttt cccagatcct caagtactcg gagtccgtcc aattcccgga caaccctccc 1380
   aacaagtacg tcgttgataa cctgaacctc atcgtgaaca agaagagcac tctgacatac 1440
   aagtactcgt ccgtcgctaa cctgctcttc aacaactaca agtaccacga caacatcgct 1500
   tctaacaaca acgccgagaa cctcaagaag gtcaagaagg aagacggaag catgcacatc 1560
   gttgagcagt acttgactca aaacgtcgat aacgttaagg gtcacaactt catcgtgttg 1620
   tccttcaaga acgaggaaag gctgaccatc gctaagaaga acaaggagtt ctactggatc 1680
   tctggcgaaa tcaaggacgt tgatgtgagc caggtcatcc aaaagtacaa cagattcaag 1740
   caccacatgt tcgtgatcgg caaggtcaac cgtcgcgagt caactacact gcacaacaac 1800
   ttgctgaagc tcttggcctt gatcctgcag ggactggtgc cactctccga cgccatcaca 1860
   ttcgccgagc aaaagctcaa ctgcaagtac aagaagttcg agttcaacta a 1911
<210> 2
   <211> 1749
   <212> DNA
   <213> Autographa californica nucleopolyhedrovirus
<400> 2
   atgactcaaa tcaacttcaa cgcttcctac acctctgcca gcactccctc tcgtgctagc     60
   ttcgacaact catactcgga gttctgcgac aagcaaccta acgattactt gtcttactac    120
   aaccacccaa ccccggacgg agctgatact gtcatctccg actctgaaac cgctgccgct    180
   agcaacttcc tcgcctcagt taactcgctc actgacaacg atttggtgga gtgtctgctc    240
   aagaccactg acaacctgga ggaagctgtg tcctctgcct actacagcga gtcactcgaa    300
   cagccagtgg tcgaacaacc ctctcctagc tcagcttacc acgccgagtc cttcgaacac    360
   tctgctggtg tcaaccagcc gtcggccaca ggcaccaaga ggaagttgga cgagtacctg    420
   gataactccc agggagttgt gggtcaattc aacaagatca agttgagacc taagtacaag    480
   aagagcacca tccagtcatg cgctacactg gaacaaacca tcaaccacaa cactaacatc    540
   tgtacagtgg cttccaccca ggagatcact cactacttca caaacgactt cgccccctac    600
   ctgatgaggt tcgacgataa cgactacaac tcgaacagat tctccgatca catgtctgaa    660
   accggttact acatgttcgt cgttaagaag tccgaggtga agcctttcga aatcatcttc    720
   gccaagtacg tctctaacgt ggtctacgag tacacaaaca actactacat ggttgacaac    780
   cgtgtgttcg ttgtgacctt cgataagatc cgcttcatga tcagctacaa cctggttaag    840
   gagactggca tcgaaatccc acactcacag gacgtctgca acgatgagac cgccgctcaa    900
   aactgcaaga agtgtcactt cgtggacgtc caccacacat tcaaggccgc tctgacctcc    960
   tacttcaacc tcgatatgta ctacgctcag acaaccttcg tgaccttgct gcaatcactc   1020
   ggcgagcgta agtgtggatt cctcttgtcg aagttgtacg agatgtacca ggacaagaac   1080
   ctcttcactt tgcccatcat gctgagccgc aaggaatcaa acgagatcga aaccgcctct   1140
   aacaacttct tcgtctcgcc atacgtttcc cagatcctca agtactcgga gtccgtccaa   1200
   ttcccggaca accctcccaa caagtacgtc gttgataacc tgaacctcat cgtgaacaag   1260
   aagagcactc tgacatacaa gtactcgtcc gtcgctaacc tgctcttcaa caactacaag   1320
   taccacgaca acatcgcttc taacaacaac gccgagaacc tcaagaaggt caagaaggaa   1380
   gacggaagca tgcacatcgt tgagcagtac ttgactcaaa acgtcgataa cgttaagggt   1440
   cacaacttca tcgtgttgtc cttcaagaac gaggaaaggc tgaccatcgc taagaagaac   1500
   aaggagttct actggatctc tggcgaaatc aaggacgttg atgtgagcca ggtcatccaa   1560
   aagtacaaca gattcaagca ccacatgttc gtgatcggca aggtcaaccg tcgcgagtca   1620
   actacactgc acaacaactt gctgaagctc ttggccttga tcctgcaggg actggtgcca   1680
   ctctccgacg ccatcacatt cgccgagcaa aagctcaact gcaagtacaa gaagttcgag   1740
   ttcaactaa                                                           1749
<210> 3
   <211> 1911
   <212> DNA
   <213> Autographa californica nucleopolyhedrovirus
<400> 3
   atgatccgta catccagcca cgtcctgaac gtccaagaaa acatcatgac ttccaactgt     60
   gcttccagcc cctactcctg tgaggccact tcagcctgcg ctgaggccca gcaactgcag    120
   gtggacacag gtggcgataa gatcgtgaac aaccaggtca ccatgactca aatcaacttc    180
   aacgcttcct acacctctgc cagcactccc tctcgtgcta gcttcgacaa ctcatactcg    240
   gagttctgcg acaagcaacc taacgattac ttgtcttact acaaccaccc aaccccggac    300
   ggagctgata ctgtcatctc cgactctgaa accgctgccg ctagcaactt cctcgcctca    360
   gttaactcgc tcactgacaa cgatttggtg gagtgtctgc tcaagaccac tgacaacctg    420
   gaggaagctg tgtcctctgc ctactacagc gagtcactcg aacagccagt ggtcgaacaa    480
   ccctctccta gctcagctta ccacgccgag tccttcgaac actctgctgg tgtcaaccag    540
   ccgtcggcca caggcaccaa gaggaagttg gacgagtacc tggataactc ccagggagtt    600
   gtgggtcaat tcaacaagat caagttgaga cctaagtaca agaagagcac catccagtca    660
   tgcgctacac tggaacaaac catcaaccac aacactaaca tctgtacagt ggcttccacc    720
   caggagatca ctcactactt cacaaacgac ttcgccccct acctgatgag gttcgacgat      780
   aacgactaca actcgaacag attctccgat cacatgtctg aaaccggtta ctacatgttc      840
   gtcgttaaga agtccgaggt gaagcctttc gaaatcatct tcgccaagta cgtctctaac      900
   gtggtctacg agtacacaaa caactactac atggttgaca accgtgtgtt cgttgtgacc      960
   ttcgataaga tccgcttcat gatcagctac aacctggtta aggagactgg catcgaaatc     1020
   ccacactcac aggacgtctg caacgatgag accgccgctc aaaactgcaa gaagtgtcac     1080
   ttcgtggacg tccaccacac attcaaggcc gctctgacct cctacttcaa cctcgatatg     1140
   tactacgctc agacaacctt cgtgaccttg ctgcaatcac tcggcgagcg taagtgtgga     1200
   ttcctcttgt cgaagttgta cgagatgtac caggacaaga acctcttcac tttgcccatc     1260
   atgctgagcc gcaaggaatc aaacgagatc gaaaccgcct ctaacaactt cttcgtctcg     1320
   ccatacgttt cccagatcct caagtactcg gagtccgtcc aattcccgga caaccctccc     1380
   aacaagtacg tcgttgataa cctgaacctc atcgtgaaca agaagagcac tctgacatac     1440
   aagtactcgt ccgtcgctaa cctgctcttc aacaactaca agtaccacga caacatcgct     1500
   tctaacaaca acgccgagaa cctcaagaag gtcaagaagg aagacggaag catgcacatc     1560
   gttgagcagt acttgactca aaacgtcgat aacgttaagg gtcacaactt catcgtgttg     1620
   tccttcaaga acgaggaaag gctgaccatc gctaagaaga acaaggagtt ctactggatc     1680
   tctggcgaaa tcaaggacgt tgatgtgagc caggtcatcc aaaagtacaa cagattcaag     1740
   caccacatgt tcgtgatcgg caaggtcaac cgtcgcgagt caactacact gcacaacaac     1800
   ttgctgaagc tcttggcctt gatcctgcag ggactggtgc cactctccga cgccatcaca     1860
   ttcgccgagc aaaagctcaa ctgcaagtac aagaagttcg agttcaacta a              1911
<210> 4
   <211> 666
   <212> DNA
   <213> Autographa californica nucleopolyhedrovirus
<400> 4
   atgactcaaa tcaacttcaa cgcttcctac acctctgcca gcactccctc tcgtgctagc 60
   ttcgacaact catactcgga gttctgcgac aagcaaccta acgattactt gtcttactac 120
   aaccacccaa ccccggacgg agctgatact gtcatctccg actctgaaac cgctgccgct 180
   agcaacttcc tcgcctcagt taactcgctc actgacaacg atttggtgga gtgtctgctc 240
   aagaccactg acaacctgga ggaagctgtg tcctctgcct actacagcga gtcactcgaa 300
   cagccagtgg tcgaacaacc ctctcctagc tcagcttacc acgccgagtc cttcgaacac 360
   tctgctggtg tcaaccagcc gtcggccaca ggcaccaaga ggaagttgga cgagtacctg 420
   gataactccc agggagttgt gggtcaattc aacaagatca agttgagacc taagtacaag 480
   aagagcacca tccagtcatg cgctacactg gaacaaacca tcaaccacaa cactaacatc 540
   tgtacagtgg cttccaccca ggagatcact cactacttca caaacgactt cgccccctac 600
   ctgatgaggt tcgacgataa cgactacaac tcgaacagat tctccgatca catgtctgaa 660
   accggt 666
<210> 5
   <211> 828
   <212> DNA
   <213> Autographa californica nucleopolyhedrovirus
<400> 5
   atgatccgta catccagcca cgtcctgaac gtccaagaaa acatcatgac ttccaactgt 60
   gcttccagcc cctactcctg tgaggccact tcagcctgcg ctgaggccca gcaactgcag 120
   gtggacacag gtggcgataa gatcgtgaac aaccaggtca ccatgactca aatcaacttc 180
   aacgcttcct acacctctgc cagcactccc tctcgtgcta gcttcgacaa ctcatactcg 240
   gagttctgcg acaagcaacc taacgattac ttgtcttact acaaccaccc aaccccggac 300
   ggagctgata ctgtcatctc cgactctgaa accgctgccg ctagcaactt cctcgcctca 360
   gttaactcgc tcactgacaa cgatttggtg gagtgtctgc tcaagaccac tgacaacctg 420
   gaggaagctg tgtcctctgc ctactacagc gagtcactcg aacagccagt ggtcgaacaa 480
   ccctctccta gctcagctta ccacgccgag tccttcgaac actctgctgg tgtcaaccag 540
   ccgtcggcca caggcaccaa gaggaagttg gacgagtacc tggataactc ccagggagtt 600
   gtgggtcaat tcaacaagat caagttgaga cctaagtaca agaagagcac catccagtca 660
   tgcgctacac tggaacaaac catcaaccac aacactaaca tctgtacagt ggcttccacc 720
   caggagatca ctcactactt cacaaacgac ttcgccccct acctgatgag gttcgacgat 780
   aacgactaca actcgaacag attctccgat cacatgtctg aaaccggt 828
<210> 6
   <211> 582
   <212> PRT
   <213> Autographa californica nucleopolyhedrovirus
<400> 6
   Met Thr Gln Ile Asn Phe Asn Ala Ser Tyr Thr Ser Ala Ser Thr Pro
   1               5                   10                  15
   Ser Arg Ala Ser Phe Asp Asn Ser Tyr Ser Glu Phe Cys Asp Lys Gln
               20                  25                  30
   Pro Asn Asp Tyr Leu Ser Tyr Tyr Asn His Pro Thr Pro Asp Gly Ala
           35                  40                  45
   Asp Thr Val Ile Ser Asp Ser Glu Thr Ala Ala Ala Ser Asn Phe Leu
       50                  55                  60
   Ala Ser Val Asn Ser Leu Thr Asp Asn Asp Leu Val Glu Cys Leu Leu
   65                  70                  75                  80
   Lys Thr Thr Asp Asn Leu Glu Glu Ala Val Ser Ser Ala Tyr Tyr Ser
                   85                  90                  95
   Glu Ser Leu Glu Gln Pro Val Val Glu Gln Pro Ser Pro Ser Ser Ala
               100                 105                 110
   Tyr His Ala Glu Ser Phe Glu His Ser Ala Gly Val Asn Gln Pro Ser
           115                 120                 125
   Ala Thr Gly Thr Lys Arg Lys Leu Asp Glu Tyr Leu Asp Asn Ser Gln
       130                 135                 140
   Gly Val Val Gly Gln Phe Asn Lys Ile Lys Leu Arg Pro Lys Tyr Lys
   145                 150                 155                 160
   Lys Ser Thr Ile Gln Ser Cys Ala Thr Leu Glu Gln Thr Ile Asn His
                   165                 170                 175
   Asn Thr Asn Ile Cys Thr Val Ala Ser Thr Gln Glu Ile Thr His Tyr
               180                 185                 190
   Phe Thr Asn Asp Phe Ala Pro Tyr Leu Met Arg Phe Asp Asp Asn Asp
           195                 200                 205
   Tyr Asn Ser Asn Arg Phe Ser Asp His Met Ser Glu Thr Gly Tyr Tyr
       210                 215                 220
   Met Phe Val Val Lys Lys Ser Glu Val Lys Pro Phe Glu Ile Ile Phe
   225                 230                 235                 240
   Ala Lys Tyr Val Ser Asn Val Val Tyr Glu Tyr Thr Asn Asn Tyr Tyr
                   245                 250                 255
   Met Val Asp Asn Arg Val Phe Val Val Thr Phe Asp Lys Ile Arg Phe
               260                 265                 270
   Met Ile Ser Tyr Asn Leu Val Lys Glu Thr Gly Ile Glu Ile Pro His
           275                 280                 285
   Ser Gln Asp Val Cys Asn Asp Glu Thr Ala Ala Gln Asn Cys Lys Lys
       290                 295                 300
   Cys His Phe Val Asp Val His His Thr Phe Lys Ala Ala Leu Thr Ser
   305 310 315 320
   Tyr Phe Asn Leu Asp Met Tyr Tyr Ala Gln Thr Thr Phe Val Thr Leu
   325 330 335
   Leu Gln Ser Leu Gly Glu Arg Lys Cys Gly Phe Leu Leu Ser Lys Leu
   340 345 350
   Tyr Glu Met Tyr Gln Asp Lys Asn Leu Phe Thr Leu Pro Ile Met Leu
   355 360 365
   Ser Arg Lys Glu Ser Asn Glu Ile Glu Thr Ala Ser Asn Asn Phe Phe
   370 375 380
   Val Ser Pro Tyr Val Ser Gln Ile Leu Lys Tyr Ser Glu Ser Val Gln
   385 390 395 400
   Phe Pro Asp Asn Pro Pro Asn Lys Tyr Val Val Asp Asn Leu Asn Leu
   405 410 415
   Ile Val Asn Lys Lys Ser Thr Leu Thr Tyr Lys Tyr Ser Ser Val Ala
   420 425 430
   Asn Leu Leu Phe Asn Asn Tyr Lys Tyr His Asp Asn Ile Ala Ser Asn
   435 440 445
   Asn Asn Ala Glu Asn Leu Lys Lys Val Lys Lys Glu Asp Gly Ser Met
   450 455 460
   His Ile Val Glu Gln Tyr Leu Thr Gln Asn Val Asp Asn Val Lys Gly
   465 470 475 480
   His Asn Phe Ile Val Leu Ser Phe Lys Asn Glu Glu Arg Leu Thr Ile
   485 490 495
   Ala Lys Lys Asn Lys Glu Phe Tyr Trp Ile Ser Gly Glu Ile Lys Asp
   500 505 510
   Val Asp Val Ser Gln Val Ile Gln Lys Tyr Asn Arg Phe Lys His His
   515 520 525
   Met Phe Val Ile Gly Lys Val Asn Arg Arg Glu Ser Thr Thr Leu His
   530 535 540
   Asn Asn Leu Leu Lys Leu Leu Ala Leu Ile Leu Gln Gly Leu Val Pro
   545 550 555 560
   Leu Ser Asp Ala Ile Thr Phe Ala Glu Gln Lys Leu Asn Cys Lys Tyr
   565 570 575
   Lys Lys Phe Glu Phe Asn
   580
<210> 7
   <211> 636
   <212> PRT
   <213> Autographa californica nucleopolyhedrovirus
<400> 7
   Met Ile Arg Thr Ser Ser His Val Leu Asn Val Gln Glu Asn Ile Met
   1               5                   10                  15
   Thr Ser Asn Cys Ala Ser Ser Pro Tyr Ser Cys Glu Ala Thr Ser Ala
               20                  25                  30
   Cys Ala Glu Ala Gln Gln Leu Gln Val Asp Thr Gly Gly Asp Lys Ile
           35                  40                  45
   Val Asn Asn Gln Val Thr Met Thr Gln Ile Asn Phe Asn Ala Ser Tyr
       50                  55                  60
   Thr Ser Ala Ser Thr Pro Ser Arg Ala Ser Phe Asp Asn Ser Tyr Ser
   65                  70                  75                  80
   Glu Phe Cys Asp Lys Gln Pro Asn Asp Tyr Leu Ser Tyr Tyr Asn His
                   85                  90                  95
   Pro Thr Pro Asp Gly Ala Asp Thr Val Ile Ser Asp Ser Glu Thr Ala
               100                 105                 110
   Ala Ala Ser Asn Phe Leu Ala Ser Val Asn Ser Leu Thr Asp Asn Asp
           115                 120                 125
   Leu Val Glu Cys Leu Leu Lys Thr Thr Asp Asn Leu Glu Glu Ala Val
       130                 135                 140
   Ser Ser Ala Tyr Tyr Ser Glu Ser Leu Glu Gln Pro Val Val Glu Gln
   145                 150                 155                 160
   Pro Ser Pro Ser Ser Ala Tyr His Ala Glu Ser Phe Glu His Ser Ala
                   165                 170                 175
   Gly Val Asn Gln Pro Ser Ala Thr Gly Thr Lys Arg Lys Leu Asp Glu
   180 185 190
   Tyr Leu Asp Asn Ser Gln Gly Val Val Gly Gln Phe Asn Lys Ile Lys
   195 200 205
   Leu Arg Pro Lys Tyr Lys Lys Ser Thr Ile Gln Ser Cys Ala Thr Leu
   210 215 220
   Glu Gln Thr Ile Asn His Asn Thr Asn Ile Cys Thr Val Ala Ser Thr
   225 230 235 240
   Gln Glu Ile Thr His Tyr Phe Thr Asn Asp Phe Ala Pro Tyr Leu Met
   245 250 255
   Arg Phe Asp Asp Asn Asp Tyr Asn Ser Asn Arg Phe Ser Asp His Met
   260 265 270
   Ser Glu Thr Gly Tyr Tyr Met Phe Val Val Lys Lys Ser Glu Val Lys
   275 280 285
   Pro Phe Glu Ile Ile Phe Ala Lys Tyr Val Ser Asn Val Val Tyr Glu
   290 295 300
   Tyr Thr Asn Asn Tyr Tyr Met Val Asp Asn Arg Val Phe Val Val Thr
   305 310 315 320
   Phe Asp Lys Ile Arg Phe Met Ile Ser Tyr Asn Leu Val Lys Glu Thr
   325 330 335
   Gly Ile Glu Ile Pro His Ser Gln Asp Val Cys Asn Asp Glu Thr Ala
   340 345 350
   Ala Gln Asn Cys Lys Lys Cys His Phe Val Asp Val His His Thr Phe
   355 360 365
   Lys Ala Ala Leu Thr Ser Tyr Phe Asn Leu Asp Met Tyr Tyr Ala Gln
   370 375 380
   Thr Thr Phe Val Thr Leu Leu Gln Ser Leu Gly Glu Arg Lys Cys Gly
   385 390 395 400
   Phe Leu Leu Ser Lys Leu Tyr Glu Met Tyr Gln Asp Lys Asn Leu Phe
   405 410 415
   Thr Leu Pro Ile Met Leu Ser Arg Lys Glu Ser Asn Glu Ile Glu Thr
   420 425 430
   Ala Ser Asn Asn Phe Phe Val Ser Pro Tyr Val Ser Gln Ile Leu Lys
   435 440 445
   Tyr Ser Glu Ser Val Gln Phe Pro Asp Asn Pro Pro Asn Lys Tyr Val
   450 455 460
   Val Asp Asn Leu Asn Leu Ile Val Asn Lys Lys Ser Thr Leu Thr Tyr
   465 470 475 480
   Lys Tyr Ser Ser Val Ala Asn Leu Leu Phe Asn Asn Tyr Lys Tyr His
   485 490 495
   Asp Asn Ile Ala Ser Asn Asn Asn Ala Glu Asn Leu Lys Lys Val Lys
   500 505 510
   Lys Glu Asp Gly Ser Met His Ile Val Glu Gln Tyr Leu Thr Gln Asn
   515 520 525
   Val Asp Asn Val Lys Gly His Asn Phe Ile Val Leu Ser Phe Lys Asn
   530 535 540
   Glu Glu Arg Leu Thr Ile Ala Lys Lys Asn Lys Glu Phe Tyr Trp Ile
   545 550 555 560
   Ser Gly Glu Ile Lys Asp Val Asp Val Ser Gln Val Ile Gln Lys Tyr
   565 570 575
   Asn Arg Phe Lys His His Met Phe Val Ile Gly Lys Val Asn Arg Arg
   580 585 590
   Glu Ser Thr Thr Leu His Asn Asn Leu Leu Lys Leu Leu Ala Leu Ile
   595 600 605
   Leu Gln Gly Leu Val Pro Leu Ser Asp Ala Ile Thr Phe Ala Glu Gln
   610 615 620
   Lys Leu Asn Cys Lys Tyr Lys Lys Phe Glu Phe Asn
   625 630 635
<210> 8
   <211> 222
   <212> PRT
   <213> Autographa californica nucleopolyhedrovirus
<400> 8
   Met Thr Gln Ile Asn Phe Asn Ala Ser Tyr Thr Ser Ala Ser Thr Pro
   1 5 10 15
   Ser Arg Ala Ser Phe Asp Asn Ser Tyr Ser Glu Phe Cys Asp Lys Gln
   20 25 30
   Pro Asn Asp Tyr Leu Ser Tyr Tyr Asn His Pro Thr Pro Asp Gly Ala
   35 40 45
   Asp Thr Val Ile Ser Asp Ser Glu Thr Ala Ala Ala Ser Asn Phe Leu
   50 55 60
   Ala Ser Val Asn Ser Leu Thr Asp Asn Asp Leu Val Glu Cys Leu Leu
   65 70 75 80
   Lys Thr Thr Asp Asn Leu Glu Glu Ala Val Ser Ser Ala Tyr Tyr Ser
   85 90 95
   Glu Ser Leu Glu Gln Pro Val Val Glu Gln Pro Ser Pro Ser Ser Ala
   100 105 110
   Tyr His Ala Glu Ser Phe Glu His Ser Ala Gly Val Asn Gln Pro Ser
   115 120 125
   Ala Thr Gly Thr Lys Arg Lys Leu Asp Glu Tyr Leu Asp Asn Ser Gln
   130 135 140
   Gly Val Val Gly Gln Phe Asn Lys Ile Lys Leu Arg Pro Lys Tyr Lys
   145 150 155 160
   Lys Ser Thr Ile Gln Ser Cys Ala Thr Leu Glu Gln Thr Ile Asn His
   165 170 175
   Asn Thr Asn Ile Cys Thr Val Ala Ser Thr Gln Glu Ile Thr His Tyr
   180 185 190
   Phe Thr Asn Asp Phe Ala Pro Tyr Leu Met Arg Phe Asp Asp Asn Asp
   195 200 205
   Tyr Asn Ser Asn Arg Phe Ser Asp His Met Ser Glu Thr Gly
   210 215 220
<210> 9
   <211> 276
   <212> PRT
   <213> Autographa californica nucleopolyhedrovirus
<400> 9
   Met Ile Arg Thr Ser Ser His Val Leu Asn Val Gln Glu Asn Ile Met
   1               5                   10                  15
   Thr Ser Asn Cys Ala Ser Ser Pro Tyr Ser Cys Glu Ala Thr Ser Ala
   20 25 30
   Cys Ala Glu Ala Gln Gln Leu Gln Val Asp Thr Gly Gly Asp Lys Ile
   35 40 45
   Val Asn Asn Gln Val Thr Met Thr Gln Ile Asn Phe Asn Ala Ser Tyr
   50 55 60
   Thr Ser Ala Ser Thr Pro Ser Arg Ala Ser Phe Asp Asn Ser Tyr Ser
   65 70 75 80
   Glu Phe Cys Asp Lys Gln Pro Asn Asp Tyr Leu Ser Tyr Tyr Asn His
   85 90 95
   Pro Thr Pro Asp Gly Ala Asp Thr Val Ile Ser Asp Ser Glu Thr Ala
   100 105 110
   Ala Ala Ser Asn Phe Leu Ala Ser Val Asn Ser Leu Thr Asp Asn Asp
   115 120 125
   Leu Val Glu Cys Leu Leu Lys Thr Thr Asp Asn Leu Glu Glu Ala Val
   130 135 140
   Ser Ser Ala Tyr Tyr Ser Glu Ser Leu Glu Gln Pro Val Val Glu Gln
   145 150 155 160
   Pro Ser Pro Ser Ser Ala Tyr His Ala Glu Ser Phe Glu His Ser Ala
   165 170 175
   Gly Val Asn Gln Pro Ser Ala Thr Gly Thr Lys Arg Lys Leu Asp Glu
   180 185 190
   Tyr Leu Asp Asn Ser Gln Gly Val Val Gly Gln Phe Asn Lys Ile Lys
   195 200 205
   Leu Arg Pro Lys Tyr Lys Lys Ser Thr Ile Gln Ser Cys Ala Thr Leu
   210 215 220
   Glu Gln Thr Ile Asn His Asn Thr Asn Ile Cys Thr Val Ala Ser Thr
   225 230 235 240
   Gln Glu Ile Thr His Tyr Phe Thr Asn Asp Phe Ala Pro Tyr Leu Met
   245 250 255
   Arg Phe Asp Asp Asn Asp Tyr Asn Ser Asn Arg Phe Ser Asp His Met
   260 265 270
   Ser Glu Thr Gly
   275
<210> 10
   <211> 128
   <212> DNA
   <213> Autographa californica nucleopolyhedrovirus
<400> 10
   atcatggaga taattaaaat gataaccatc tcgcaaataa ataagtattt tactgttttc 60
   gtaacagttt tgtaataaaa aaacctataa atattccgga ttattcatac cgtcccacca 120
   tcgggcgc 128
<210> 11
   <211> 122
   <212> DNA
   <213> Autographa californica nucleopolyhedrovirus
<400> 11
   atacggacct ttaattcaac ccaacacaat atattatagt taaataagaa ttattatcaa 60
   atcatttgta tattaattaa aatactatac tgtaaattac attttattta caatcactcg 120
   ac 122
<210> 12
   <211> 571
   <212> DNA
   <213> Artificial Sequence
<220>
   <223> Recombinant chimeric promoter
<400> 12
   aaaaacatcg attagggtga ctgaaggtta cattggggta ggttatggtt aatacgtaat 60
   ggtttaacac caaaacgata tcatggattt tatataaggt gtaataatat ttttaatgag 120
   tggacgcgtc gggtcaatgt cctgcctatt gacgtcataa catattaggt gattatatta 180
   aaaatagttt aaactcaaat attacttgca agtttaagtt tcatcataat ctgatcataa 240
   gtttcaccca aacagaaacc aaaagcataa ctatcgaata tctttagctt cccatgaaga 300
   aagattaccg taaccatcac taggatttta tacgattgta gaaaataaag tattctcagt 360
   ctcttttcag agcgctataa aaaggggtgc attctcggta agagtacagt tgaactcaca 420
   tcgagttaac tccacgctgc agtctcgaga tacggacctt taattcaacc caacacaata 480
   tattatagtt aaataagaat tattatcaaa tcatttgtat attaattaaa atactatact 540
   gtaaattaca ttttatttac aatcactcga c 571
<210> 13
   <211> 465
   <212> DNA
   <213> Artificial Sequence
<220>
   <223> Recombinant chimeric promoter
<400> 13
   ggtaccaaat tccgttttgc gacgatgcag agtttttgaa caggctgctc aaacacatag 60
   atccgtaccc gctcagtcgg atgtattaca atgcagccaa taccatgttt tacacgacta 120
   tggaaaacta tgccgtgtcc aattgcaagt tcaacattga ggattacaat aacatattta 180
   aggtgatgga aaatattagg aaacacagca acaaaaattc aaacgaccaa gacgagttaa 240
   acatatattt gggagttcag tcgtcgaatg caaagcgtaa aaaatattaa taaggtaaaa 300
   attacagcta cataaattac acaatttaaa ctgcagtctg gagatacgga cctttaattc 360
   aacccaacac aatatattat agttaaataa gaattattat caaatcattt gtatattaat 420
   taaaatacta tactgtaaat tacattttat ttacaatcac tcgac 465
<210> 14
   <211> 436
   <212> DNA
   <213> Autographa californica nucleopolyhedrovirus
<400> 14
   aaaaacatcg attagggtga ctgaaggtta cattggggta ggttatggtt aatacgtaat 60
   ggtttaacac caaaacgata tcatggattt tatataaggt gtaataatat ttttaatgag 120
   tggacgcgtc gggtcaatgt cctgcctatt gacgtcataa catattaggt gattatatta 180
   aaaatagttt aaactcaaat attacttgca agtttaagtt tcatcataat ctgatcataa 240
   gtttcaccca aacagaaacc aaaagcataa ctatcgaata tctttagctt cccatgaaga 300
   aagattaccg taaccatcac taggatttta tacgattgta gaaaataaag tattctcagt 360
   ctcttttcag agcgctataa aaaggggtgc attctcggta agagtacagt tgaactcaca 420
   tcgagttaac tccacg 436
<210> 15
   <211> 1176
   <212> DNA
   <213> Artificial Sequence
<220>
   <223> Recombinant chimeric promoter
<400> 15
   cttgaatgtt agtgaaaccc cctgcgacac aagtattaca ttccttagtg cttgaatcct 60
   ttaggaaaga aaagccaatt ttcaaaatct tagcacttgt taactcgcga aaaagaccaa 120
   cagatttccc atactacaat tcgacattag aaatgtaaac ccattatcat tatttacgcc 180
   tcatttccat ccaataataa gtttaagtac gttgagataa aactggctta cctagaactt 240
   gacatggcga cctcttgcac tctgtatctc aagtcaactt tctctatcca aatatttgat 300
   aacatttgac atgatattga agtaagattg ttactaaggc ttacattgta atattactga 360
   cgcaagttct ttatcaataa aatagctgaa aacaaaaaaa aaaacatcga ttagggtgac 420
   tgaaggttac attggggtag gttatggtta atacgtaatg gtttaacacc aaaacgatat 480
   catggattga ctttataaat tttatataag gtgtaataat atttttaatg agtggacgcg 540
   tcgggtcaat gtcctgccta ttgacgtcat aacatattag gtgattatat taaaaatact 600
   caaatattac ttgcaagttt aagtttcatc ataatctgat cataagtttc acccaaacag 660
   aaaccaaaag cataactatc tgctatttga atatctttag cttcccatga agaaagatta 720
   ccgtaaccat cactaggatt ttatacgatt gtagaaaata aagtattctc agtctctttt 780
   cagtttaaaa tctgctggca tttttacaag tcgctgtatc agtcaatgtt tatacaatat 840
   gtcaatgtac tttcgtatta atcagaaaaa aatattctac tagttttgat aagctatcac 900
   ttttgttaca ttgtactgcc ctttacagtt catcaggtat ttatgaatga catattggag 960
   aaacatcgta atcagtccag tataaaaagg ggtgcattct cggtaagagt acagttgaac 1020
   tcacatcgag ttaactccac gctgcagtct cgagatacgg acctttaatt caacccaaca 1080
   caatatatta tagttaaata agaattatta tcaaatcatt tgtatattaa ttaaaatact 1140
   atactgtaaa ttacatttta tttacaatca ctcgac 1176
<210> 16
   <211> 250
   <212> DNA
   <213> Artificial Sequence
<220>
   <223> Recombinant chimeric promoter
<400> 16
   atcatggaga taattaaaat gataaccatc tcgcaaataa ataagtattt tactgttttc 60
   gtaacagttt tgtaataaaa aaacctataa atattccgga ttattcatac cgtcccacca 120
   tcgggcgcat acggaccttt aattcaaccc aacacaatat attatagtta aataagaatt 180
   attatcaaat catttgtata ttaattaaaa tactatactg taaattacat tttatttaca 240
   atcactcgac 250
<210> 17
   <211> 3163
   <212> DNA
   <213> Artificial Sequence
<220>
   <223> Recombinant expression cassette
<400> 17
   ttagttgaac tcgaacttct tgtacttgca gttgagcttt tgctcggcga atgtgatggc 60
   gtcggagagt ggcaccagtc cctgcaggat caaggccaag agcttcagca agttgttgtg 120
   cagtgtagtt gactcgcgac ggttgacctt gccgatcacg aacatgtggt gcttgaatct 180
   gttgtacttt tggatgacct ggctcacatc aacgtccttg atttcgccag agatccagta 240
   gaactccttg ttcttcttag cgatggtcag cctttcctcg ttcttgaagg acaacacgat 300
   gaagttgtga cccttaacgt tatcgacgtt ttgagtcaag tactgctcaa cgatgtgcat 360
   gcttccgtct tccttcttga ccttcttgag gttctcggcg ttgttgttag aagcgatgtt 420
   gtcgtggtac ttgtagttgt tgaagagcag gttagcgacg gacgagtact tgtatgtcag 480
   agtgctcttc ttgttcacga tgaggttcag gttatcaacg acgtacttgt tgggagggtt 540
   gtccgggaat tggacggact ccgagtactt gaggatctgg gaaacgtatg gcgagacgaa 600
   gaagttgtta gaggcggttt cgatctcgtt tgattccttg cggctcagca tgatgggcaa 660
   agtgaagagg ttcttgtcct ggtacatctc gtacaacttc gacaagagga atccacactt 720
   acgctcgccg agtgattgca gcaaggtcac gaaggttgtc tgagcgtagt acatatcgag 780
   gttgaagtag gaggtcagag cggccttgaa tgtgtggtgg acgtccacga agtgacactt 840
   cttgcagttt tgagcggcgg tctcatcgtt gcagacgtcc tgtgagtgtg ggatttcgat 900
   gccagtctcc ttaaccaggt tgtagctgat catgaagcgg atcttatcga aggtcacaac 960
   gaacacacgg ttgtcaacca tgtagtagtt gtttgtgtac tcgtagacca cgttagagac 1020
   gtacttggcg aagatgattt cgaaaggctt cacctcggac ttcttaacga cgaacatgta 1080
   gtaaccggtt tcagacatgt gatcggagaa tctgttcgag ttgtagtcgt tatcgtcgaa 1140
   cctcatcagg tagggggcga agtcgtttgt gaagtagtga gtgatctcct gggtggaagc 1200
   cactgtacag atgttagtgt tgtggttgat ggtttgttcc agtgtagcgc atgactggat 1260
   ggtgctcttc ttgtacttag gtctcaactt gatcttgttg aattgaccca caactccctg 1320
   ggagttatcc aggtactcgt ccaacttcct cttggtgcct gtggccgacg gctggttgac 1380
   accagcagag tgttcgaagg actcggcgtg gtaagctgag ctaggagagg gttgttcgac 1440
   cactggctgt tcgagtgact cgctgtagta ggcagaggac acagcttcct ccaggttgtc 1500
   agtggtcttg agcagacact ccaccaaatc gttgtcagtg agcgagttaa ctgaggcgag 1560
   gaagttgcta gcggcagcgg tttcagagtc ggagatgaca gtatcagctc cgtccggggt 1620
   tgggtggttg tagtaagaca agtaatcgtt aggttgcttg tcgcagaact ccgagtatga 1680
   gttgtcgaag ctagcacgag agggagtgct ggcagaggtg taggaagcgt tgaagttgat 1740
   ttgagtcatg gtgacctggt tgttcacgat cttatcgcca cctgtgtcca cctgcagttg 1800
   ctgggcctca gcgcaggctg aagtggcctc acaggagtag gggctggaag cacagttgga 1860
   agtcatgatg ttttcttgga cgttcaggac gtggctggat gtacggatca tagatctatt 1920
   gggtcatcta gattcgaaag cggccgcgac tagtgagctc gtcgacgtag gcctttgaat 1980
   tccgcgcgct tcggaccggg atccgcgccc gatggtggga cggtatgaat aatccggaat 2040
   atttataggt ttttttatta caaaactgtt acgaaaacag taaaatactt atttatttgc 2100
   gagatggtta tcattttaat tatctccatg atctattaat attccggagt atacatcgat 2160
   gttgacccca acaaaagatt tataattaat cataatcacg aacaacaaca agtcaatgaa 2220
   acaaataaac aagttgtcga taaaacattc ataaatgaca cagcaacata caattcttgc 2280
   ataataaaaa tttaaatgac atcatatttg agaataacaa atgacattat ccctcgattg 2340
   tgttttacaa gtagaattct acccgtaaag cgagtttagt tttgaaaaac aaatgacatc 2400
   atttgtataa tgacatcatc ccctgattgt gttttacaag tagaattcta tccgtaaagc 2460
   gagttcagtt ttgaaaacaa atgagtcata cctaaacacg ttaataatct tctgatatca 2520
   gcttatgact caagttatga gccgtgtgca aaacatgaga taagtttatg acatcatcca 2580
   ctgatcgtgc gttacaagta gaattctact cgtaaagcca gttcggttat gagccgtgtg 2640
   caaaacatga catcagctta tgactcatac ttgattgtgt tttacgcgta gaattctact 2700
   cgtaaagcga gttcggttat gagccgtgtg caaaacatga catcagctta tgagtcataa 2760
   ttaatcgtgc gttacaagta gaattctact cgtaaagcga gttgaaggat catatttagt 2820
   tgcgtttatg agataagatt gaaagcacgt gtaaaatgtt tcccgcgcgt tggcacaact 2880
   atttacaatg cggccaagtt ataaaagatt ctaatctgat atgttttaaa acacctttgc 2940
   ggcccgagtt gtttgcgtac gtgactagcg aagaagatgt gtggaccgca gaacagatag 3000
   taaaacaaaa ccctagtatt ggagcaataa tcgatgagct catacggacc tttaattcaa 3060
   cccaacacaa tatattatag ttaaataaga attattatca aatcatttgt atattaatta 3120
   aaatactata ctgtaaatta cattttattt acaatcactc gac 3163
<210> 18
   <211> 3656
   <212> DNA
   <213> Artificial Sequence
<220>
   <223> Recombinant expression cassette
<400> 18
   ttagttgaac tcgaacttct tgtacttgca gttgagcttt tgctcggcga atgtgatggc 60
   gtcggagagt ggcaccagtc cctgcaggat caaggccaag agcttcagca agttgttgtg 120
   cagtgtagtt gactcgcgac ggttgacctt gccgatcacg aacatgtggt gcttgaatct 180
   gttgtacttt tggatgacct ggctcacatc aacgtccttg atttcgccag agatccagta 240
   gaactccttg ttcttcttag cgatggtcag cctttcctcg ttcttgaagg acaacacgat 300
   gaagttgtga cccttaacgt tatcgacgtt ttgagtcaag tactgctcaa cgatgtgcat 360
   gcttccgtct tccttcttga ccttcttgag gttctcggcg ttgttgttag aagcgatgtt 420
   gtcgtggtac ttgtagttgt tgaagagcag gttagcgacg gacgagtact tgtatgtcag 480
   agtgctcttc ttgttcacga tgaggttcag gttatcaacg acgtacttgt tgggagggtt 540
   gtccgggaat tggacggact ccgagtactt gaggatctgg gaaacgtatg gcgagacgaa 600
   gaagttgtta gaggcggttt cgatctcgtt tgattccttg cggctcagca tgatgggcaa 660
   agtgaagagg ttcttgtcct ggtacatctc gtacaacttc gacaagagga atccacactt 720
   acgctcgccg agtgattgca gcaaggtcac gaaggttgtc tgagcgtagt acatatcgag 780
   gttgaagtag gaggtcagag cggccttgaa tgtgtggtgg acgtccacga agtgacactt 840
   cttgcagttt tgagcggcgg tctcatcgtt gcagacgtcc tgtgagtgtg ggatttcgat 900
   gccagtctcc ttaaccaggt tgtagctgat catgaagcgg atcttatcga aggtcacaac 960
   gaacacacgg ttgtcaacca tgtagtagtt gtttgtgtac tcgtagacca cgttagagac 1020
   gtacttggcg aagatgattt cgaaaggctt cacctcggac ttcttaacga cgaacatgta 1080
   gtaaccggtt tcagacatgt gatcggagaa tctgttcgag ttgtagtcgt tatcgtcgaa 1140
   cctcatcagg tagggggcga agtcgtttgt gaagtagtga gtgatctcct gggtggaagc 1200
   cactgtacag atgttagtgt tgtggttgat ggtttgttcc agtgtagcgc atgactggat 1260
   ggtgctcttc ttgtacttag gtctcaactt gatcttgttg aattgaccca caactccctg 1320
   ggagttatcc aggtactcgt ccaacttcct cttggtgcct gtggccgacg gctggttgac 1380
   accagcagag tgttcgaagg actcggcgtg gtaagctgag ctaggagagg gttgttcgac 1440
   cactggctgt tcgagtgact cgctgtagta ggcagaggac acagcttcct ccaggttgtc 1500
   agtggtcttg agcagacact ccaccaaatc gttgtcagtg agcgagttaa ctgaggcgag 1560
   gaagttgcta gcggcagcgg tttcagagtc ggagatgaca gtatcagctc cgtccggggt 1620
   tgggtggttg tagtaagaca agtaatcgtt aggttgcttg tcgcagaact ccgagtatga 1680
   gttgtcgaag ctagcacgag agggagtgct ggcagaggtg taggaagcgt tgaagttgat 1740
   ttgagtcatg gtgacctggt tgttcacgat cttatcgcca cctgtgtcca cctgcagttg 1800
   ctgggcctca gcgcaggctg aagtggcctc acaggagtag gggctggaag cacagttgga 1860
   agtcatgatg ttttcttgga cgttcaggac gtggctggat gtacggatca tagatctatt 1920
   gggtcatcta gattcgaaag cggccgcgac tagtgagctc gtcgacgtag gcctttgaat 1980
   tccgcgcgct tcggaccggg atccgcgccc gatggtggga cggtatgaat aatccggaat 2040
   atttataggt ttttttatta caaaactgtt acgaaaacag taaaatactt atttatttgc 2100
   gagatggtta tcattttaat tatctccatg atctattaat attccggagt atacatcgat 2160
   gttgacccca acaaaagatt tataattaat cataatcacg aacaacaaca agtcaatgaa 2220
   acaaataaac aagttgtcga taaaacattc ataaatgaca cagcaacata caattcttgc 2280
   ataataaaaa tttaaatgac atcatatttg agaataacaa atgacattat ccctcgattg 2340
   tgttttacaa gtagaattct acccgtaaag cgagtttagt tttgaaaaac aaatgacatc 2400
   atttgtataa tgacatcatc ccctgattgt gttttacaag tagaattcta tccgtaaagc 2460
   gagttcagtt ttgaaaacaa atgagtcata cctaaacacg ttaataatct tctgatatca 2520
   gcttatgact caagttatga gccgtgtgca aaacatgaga taagtttatg acatcatcca 2580
   ctgatcgtgc gttacaagta gaattctact cgtaaagcca gttcggttat gagccgtgtg 2640
   caaaacatga catcagctta tgactcatac ttgattgtgt tttacgcgta gaattctact 2700
   cgtaaagcga gttcggttat gagccgtgtg caaaacatga catcagctta tgagtcataa 2760
   ttaatcgtgc gttacaagta gaattctact cgtaaagcga gttgaaggat catatttagt 2820
   tgcgtttatg agataagatt gaaagcacgt gtaaaatgtt tcccgcgcgt tggcacaact 2880
   atttacaatg cggccaagtt ataaaagatt ctaatctgat atgttttaaa acacctttgc 2940
   ggcccgagtt gtttgcgtac gtgactagcg aagaagatgt gtggaccgca gaacagatag 3000
   taaaacaaaa ccctagtatt ggagcaataa tcgatgagct cgtcgacgta ggcctttgaa 3060
   ttccgcgcgc ttcggaccgg gatccaaaaa catcgattag ggtgactgaa ggttacattg 3120
   gggtaggtta tggttaatac gtaatggttt aacaccaaaa cgatatcatg gattttatat 3180
   aaggtgtaat aatattttta atgagtggac gcgtcgggtc aatgtcctgc ctattgacgt 3240
   cataacatat taggtgatta tattaaaaat agtttaaact caaatattac ttgcaagttt 3300
   aagtttcatc ataatctgat cataagtttc acccaaacag aaaccaaaag cataactatc 3360
   gaatatcttt agcttcccat gaagaaagat taccgtaacc atcactagga ttttatacga 3420
   ttgtagaaaa taaagtattc tcagtctctt ttcagagcgc tataaaaagg ggtgcattct 3480
   cggtaagagt acagttgaac tcacatcgag ttaactccac gctgcagtct cgagatacgg 3540
   acctttaatt caacccaaca caatatatta tagttaaata agaattatta tcaaatcatt 3600
   tgtatattaa ttaaaatact atactgtaaa ttacatttta tttacaatca ctcgac 3656
<210> 19
   <211> 3541
   <212> DNA
   <213> Artificial Sequence
<220>
   <223> Recombinant expression cassette
<400> 19
   ttagttgaac tcgaacttct tgtacttgca gttgagcttt tgctcggcga atgtgatggc 60
   gtcggagagt ggcaccagtc cctgcaggat caaggccaag agcttcagca agttgttgtg 120
   cagtgtagtt gactcgcgac ggttgacctt gccgatcacg aacatgtggt gcttgaatct 180
   gttgtacttt tggatgacct ggctcacatc aacgtccttg atttcgccag agatccagta 240
   gaactccttg ttcttcttag cgatggtcag cctttcctcg ttcttgaagg acaacacgat 300
   gaagttgtga cccttaacgt tatcgacgtt ttgagtcaag tactgctcaa cgatgtgcat 360
   gcttccgtct tccttcttga ccttcttgag gttctcggcg ttgttgttag aagcgatgtt 420
   gtcgtggtac ttgtagttgt tgaagagcag gttagcgacg gacgagtact tgtatgtcag 480
   agtgctcttc ttgttcacga tgaggttcag gttatcaacg acgtacttgt tgggagggtt 540
   gtccgggaat tggacggact ccgagtactt gaggatctgg gaaacgtatg gcgagacgaa 600
   gaagttgtta gaggcggttt cgatctcgtt tgattccttg cggctcagca tgatgggcaa 660
   agtgaagagg ttcttgtcct ggtacatctc gtacaacttc gacaagagga atccacactt 720
   acgctcgccg agtgattgca gcaaggtcac gaaggttgtc tgagcgtagt acatatcgag 780
   gttgaagtag gaggtcagag cggccttgaa tgtgtggtgg acgtccacga agtgacactt 840
   cttgcagttt tgagcggcgg tctcatcgtt gcagacgtcc tgtgagtgtg ggatttcgat 900
   gccagtctcc ttaaccaggt tgtagctgat catgaagcgg atcttatcga aggtcacaac 960
   gaacacacgg ttgtcaacca tgtagtagtt gtttgtgtac tcgtagacca cgttagagac 1020
   gtacttggcg aagatgattt cgaaaggctt cacctcggac ttcttaacga cgaacatgta 1080
   gtaaccggtt tcagacatgt gatcggagaa tctgttcgag ttgtagtcgt tatcgtcgaa 1140
   cctcatcagg tagggggcga agtcgtttgt gaagtagtga gtgatctcct gggtggaagc 1200
   cactgtacag atgttagtgt tgtggttgat ggtttgttcc agtgtagcgc atgactggat 1260
   ggtgctcttc ttgtacttag gtctcaactt gatcttgttg aattgaccca caactccctg 1320
   ggagttatcc aggtactcgt ccaacttcct cttggtgcct gtggccgacg gctggttgac 1380
   accagcagag tgttcgaagg actcggcgtg gtaagctgag ctaggagagg gttgttcgac 1440
   cactggctgt tcgagtgact cgctgtagta ggcagaggac acagcttcct ccaggttgtc 1500
   agtggtcttg agcagacact ccaccaaatc gttgtcagtg agcgagttaa ctgaggcgag 1560
   gaagttgcta gcggcagcgg tttcagagtc ggagatgaca gtatcagctc cgtccggggt 1620
   tgggtggttg tagtaagaca agtaatcgtt aggttgcttg tcgcagaact ccgagtatga 1680
   gttgtcgaag ctagcacgag agggagtgct ggcagaggtg taggaagcgt tgaagttgat 1740
   ttgagtcatg gtgacctggt tgttcacgat cttatcgcca cctgtgtcca cctgcagttg 1800
   ctgggcctca gcgcaggctg aagtggcctc acaggagtag gggctggaag cacagttgga 1860
   agtcatgatg ttttcttgga cgttcaggac gtggctggat gtacggatca tagatctatc 1920
   tagattcgaa agcggccgcg actagtgagc tcgtcgacgt aggcctttga attccgcgcg 1980
   cttcggaccg ggatccgcgc ccgatggtgg gacggtatga ataatccgga atatttatag 2040
   gtttttttat tacaaaactg ttacgaaaac agtaaaatac ttatttattt gcgagatggt 2100
   tatcatttta attatctcca tgatctatta atattccgga gtatacatcg atgttgaccc 2160
   caacaaaaga tttataatta atcataatca cgaacaacaa caagtcaatg aaacaaataa 2220
   acaagttgtc gataaaacat tcataaatga cacagcaaca tacaattctt gcataataaa 2280
   aatttaaatg acatcatatt tgagaataac aaatgacatt atccctcgat tgtgttttac 2340
   aagtagaatt ctacccgtaa agcgagttta gttttgaaaa acaaatgaca tcatttgtat 2400
   aatgacatca tcccctgatt gtgttttaca agtagaattc tatccgtaaa gcgagttcag 2460
   ttttgaaaac aaatgagtca tacctaaaca cgttaataat cttctgatat cagcttatga 2520
   ctcaagttat gagccgtgtg caaaacatga gataagttta tgacatcatc cactgatcgt 2580
   gcgttacaag tagaattcta ctcgtaaagc cagttcggtt atgagccgtg tgcaaaacat 2640
   gacatcagct tatgactcat acttgattgt gttttacgcg tagaattcta ctcgtaaagc 2700
   gagttcggtt atgagccgtg tgcaaaacat gacatcagct tatgagtcat aattaatcgt 2760
   gcgttacaag tagaattcta ctcgtaaagc gagttgaagg atcatattta gttgcgttta 2820
   tgagataaga ttgaaagcac gtgtaaaatg tttcccgcgc gttggcacaa ctatttacaa 2880
   tgcggccaag ttataaaaga ttctaatctg atatgtttta aaacaccttt gcggcccgag 2940
   ttgtttgcgt acgtgactag cgaagaagat gtgtggaccg cagaacagat agtaaaacaa 3000
   aaccctagta ttggagcaat aatcgatgag ctcgtcgacg taggcctttg aattccgcgc 3060
   gcttcggacc gggatcggta ccaaattccg ttttgcgacg atgcagagtt tttgaacagg 3120
   ctgctcaaac acatagatcc gtacccgctc agtcggatgt attacaatgc agccaatacc 3180
   atgttttaca cgactatgga aaactatgcc gtgtccaatt gcaagttcaa cattgaggat 3240
   tacaataaca tatttaaggt gatggaaaat attaggaaac acagcaacaa aaattcaaac 3300
   gaccaagacg agttaaacat atatttggga gttcagtcgt cgaatgcaaa gcgtaaaaaa 3360
   tattaataag gtaaaaatta cagctacata aattacacaa tttaaactgc agtctggaga 3420
   tacggacctt taattcaacc caacacaata tattatagtt aaataagaat tattatcaaa 3480
   tcatttgtat attaattaaa atactatact gtaaattaca ttttatttac aatcactcga 3540
   c 3541
<210> 20
   <211> 3512
   <212> DNA
   <213> Artificial Sequence
<220>
   <223> Recombinant expression cassette
<400> 20
   ttagttgaac tcgaacttct tgtacttgca gttgagcttt tgctcggcga atgtgatggc 60
   gtcggagagt ggcaccagtc cctgcaggat caaggccaag agcttcagca agttgttgtg 120
   cagtgtagtt gactcgcgac ggttgacctt gccgatcacg aacatgtggt gcttgaatct 180
   gttgtacttt tggatgacct ggctcacatc aacgtccttg atttcgccag agatccagta 240
   gaactccttg ttcttcttag cgatggtcag cctttcctcg ttcttgaagg acaacacgat 300
   gaagttgtga cccttaacgt tatcgacgtt ttgagtcaag tactgctcaa cgatgtgcat 360
   gcttccgtct tccttcttga ccttcttgag gttctcggcg ttgttgttag aagcgatgtt 420
   gtcgtggtac ttgtagttgt tgaagagcag gttagcgacg gacgagtact tgtatgtcag 480
   agtgctcttc ttgttcacga tgaggttcag gttatcaacg acgtacttgt tgggagggtt 540
   gtccgggaat tggacggact ccgagtactt gaggatctgg gaaacgtatg gcgagacgaa 600
   gaagttgtta gaggcggttt cgatctcgtt tgattccttg cggctcagca tgatgggcaa 660
   agtgaagagg ttcttgtcct ggtacatctc gtacaacttc gacaagagga atccacactt 720
   acgctcgccg agtgattgca gcaaggtcac gaaggttgtc tgagcgtagt acatatcgag 780
   gttgaagtag gaggtcagag cggccttgaa tgtgtggtgg acgtccacga agtgacactt 840
   cttgcagttt tgagcggcgg tctcatcgtt gcagacgtcc tgtgagtgtg ggatttcgat 900
   gccagtctcc ttaaccaggt tgtagctgat catgaagcgg atcttatcga aggtcacaac 960
   gaacacacgg ttgtcaacca tgtagtagtt gtttgtgtac tcgtagacca cgttagagac 1020
   gtacttggcg aagatgattt cgaaaggctt cacctcggac ttcttaacga cgaacatgta 1080
   gtaaccggtt tcagacatgt gatcggagaa tctgttcgag ttgtagtcgt tatcgtcgaa 1140
   cctcatcagg tagggggcga agtcgtttgt gaagtagtga gtgatctcct gggtggaagc 1200
   cactgtacag atgttagtgt tgtggttgat ggtttgttcc agtgtagcgc atgactggat 1260
   ggtgctcttc ttgtacttag gtctcaactt gatcttgttg aattgaccca caactccctg 1320
   ggagttatcc aggtactcgt ccaacttcct cttggtgcct gtggccgacg gctggttgac 1380
   accagcagag tgttcgaagg actcggcgtg gtaagctgag ctaggagagg gttgttcgac 1440
   cactggctgt tcgagtgact cgctgtagta ggcagaggac acagcttcct ccaggttgtc 1500
   agtggtcttg agcagacact ccaccaaatc gttgtcagtg agcgagttaa ctgaggcgag 1560
   gaagttgcta gcggcagcgg tttcagagtc ggagatgaca gtatcagctc cgtccggggt 1620
   tgggtggttg tagtaagaca agtaatcgtt aggttgcttg tcgcagaact ccgagtatga 1680
   gttgtcgaag ctagcacgag agggagtgct ggcagaggtg taggaagcgt tgaagttgat 1740
   ttgagtcatg gtgacctggt tgttcacgat cttatcgcca cctgtgtcca cctgcagttg 1800
   ctgggcctca gcgcaggctg aagtggcctc acaggagtag gggctggaag cacagttgga 1860
   agtcatgatg ttttcttgga cgttcaggac gtggctggat gtacggatca tagatctatc 1920
   tagatgcatt cgcgaggtac cgagctcgaa ttcactggcc gtcgttttac aacgtcgtga    1980
   ctgggaaaac cctggcgtta cccaacttaa tcgccttgca gcacatcccc ctttcgccag    2040
   ctgctagcac catggctcga gcgtggagtt aactcgatgt gagttcaact gtactcttac    2100
   cgagaatgca ccccttttta tagcgctctg aaaagagact gagaatactt tattttctac    2160
   aatcgtataa aatcctagtg atggttacgg taatctttct tcatgggaag ctaaagatat    2220
   tcgatagtta tgcttttggt ttctgtttgg gtgaaactta tgatcagatt atgatgaaac    2280
   ttaaacttgc aagtaatatt tgagtttaaa ctatttttaa tataatcacc taatatgtta    2340
   tgacgtcaat aggcaggaca ttgacccgac gcgtccactc attaaaaata ttattacacc    2400
   ttatataaaa tccatgatat cgttttggtg ttaaaccatt acgtattaac cataacctac    2460
   cccaatgtaa ccttcagtca ccctaatcga tgtttttgta tacatcgatg ttgaccccaa    2520
   caaaagattt ataattaatc ataatcacga acaacaacaa gtcaatgaaa caaataaaca    2580
   agttgtcgat aaaacattca taaatgacac agcaacatac aattcttgca taataaaaat    2640
   ttaaatgaca tcatatttga gaataacaaa tgacattatc cctcgattgt gttttacaag    2700
   tagaattcta cccgtaaagc gagtttagtt ttgaaaaaca aatgacatca tttgtataat    2760
   gacatcatcc cctgattgtg ttttacaagt agaattctat ccgtaaagcg agttcagttt    2820
   tgaaaacaaa tgagtcatac ctaaacacgt taataatctt ctgatatcag cttatgactc    2880
   aagttatgag ccgtgtgcaa aacatgagat aagtttatga catcatccac tgatcgtgcg    2940
   ttacaagtag aattctactc gtaaagccag ttcggttatg agccgtgtgc aaaacatgac    3000
   atcagcttat gactcatact tgattgtgtt ttacgcgtag aattctactc gtaaagcgag    3060
   ttcggttatg agccgtgtgc aaaacatgac atcagcttat gagtcataat taatcgtgcg    3120
   ttacaagtag aattctactc gtaaagcgag ttgaaggatc atatttagtt gcgtttatga    3180
   gataagattg aaagcacgtg taaaatgttt cccgcgcgtt ggcacaacta tttacaatgc    3240
   ggccaagtta taaaagattc taatctgata tgttttaaaa cacctttgcg gcccgagttg    3300
   tttgcgtacg tgactagcga agaagatgtg tggaccgcag aacagatagt aaaacaaaac    3360
   cctagtattg gagcaataat cgatgagctc atacggacct ttaattcaac ccaacacaat    3420
   atattatagt taaataagaa ttattatcaa atcatttgta tattaattaa aatactatac    3480
   tgtaaattac atttatttta caatcactcg ac                                  3512
<210> 21
   <211> 4005
   <212> DNA
   <213> Artificial Sequence
<220>
   <223> Recombinant expression cassette
<400> 21
   ttagttgaac tcgaacttct tgtacttgca gttgagcttt tgctcggcga atgtgatggc 60
   gtcggagagt ggcaccagtc cctgcaggat caaggccaag agcttcagca agttgttgtg 120
   cagtgtagtt gactcgcgac ggttgacctt gccgatcacg aacatgtggt gcttgaatct 180
   gttgtacttt tggatgacct ggctcacatc aacgtccttg atttcgccag agatccagta 240
   gaactccttg ttcttcttag cgatggtcag cctttcctcg ttcttgaagg acaacacgat 300
   gaagttgtga cccttaacgt tatcgacgtt ttgagtcaag tactgctcaa cgatgtgcat 360
   gcttccgtct tccttcttga ccttcttgag gttctcggcg ttgttgttag aagcgatgtt 420
   gtcgtggtac ttgtagttgt tgaagagcag gttagcgacg gacgagtact tgtatgtcag 480
   agtgctcttc ttgttcacga tgaggttcag gttatcaacg acgtacttgt tgggagggtt 540
   gtccgggaat tggacggact ccgagtactt gaggatctgg gaaacgtatg gcgagacgaa 600
   gaagttgtta gaggcggttt cgatctcgtt tgattccttg cggctcagca tgatgggcaa 660
   agtgaagagg ttcttgtcct ggtacatctc gtacaacttc gacaagagga atccacactt 720
   acgctcgccg agtgattgca gcaaggtcac gaaggttgtc tgagcgtagt acatatcgag 780
   gttgaagtag gaggtcagag cggccttgaa tgtgtggtgg acgtccacga agtgacactt 840
   cttgcagttt tgagcggcgg tctcatcgtt gcagacgtcc tgtgagtgtg ggatttcgat 900
   gccagtctcc ttaaccaggt tgtagctgat catgaagcgg atcttatcga aggtcacaac 960
   gaacacacgg ttgtcaacca tgtagtagtt gtttgtgtac tcgtagacca cgttagagac 1020
   gtacttggcg aagatgattt cgaaaggctt cacctcggac ttcttaacga cgaacatgta 1080
   gtaaccggtt tcagacatgt gatcggagaa tctgttcgag ttgtagtcgt tatcgtcgaa 1140
   cctcatcagg tagggggcga agtcgtttgt gaagtagtga gtgatctcct gggtggaagc 1200
   cactgtacag atgttagtgt tgtggttgat ggtttgttcc agtgtagcgc atgactggat 1260
   ggtgctcttc ttgtacttag gtctcaactt gatcttgttg aattgaccca caactccctg 1320
   ggagttatcc aggtactcgt ccaacttcct cttggtgcct gtggccgacg gctggttgac 1380
   accagcagag tgttcgaagg actcggcgtg gtaagctgag ctaggagagg gttgttcgac 1440
   cactggctgt tcgagtgact cgctgtagta ggcagaggac acagcttcct ccaggttgtc 1500
   agtggtcttg agcagacact ccaccaaatc gttgtcagtg agcgagttaa ctgaggcgag 1560
   gaagttgcta gcggcagcgg tttcagagtc ggagatgaca gtatcagctc cgtccggggt 1620
   tgggtggttg tagtaagaca agtaatcgtt aggttgcttg tcgcagaact ccgagtatga 1680
   gttgtcgaag ctagcacgag agggagtgct ggcagaggtg taggaagcgt tgaagttgat 1740
   ttgagtcatg gtgacctggt tgttcacgat cttatcgcca cctgtgtcca cctgcagttg 1800
   ctgggcctca gcgcaggctg aagtggcctc acaggagtag gggctggaag cacagttgga 1860
   agtcatgatg ttttcttgga cgttcaggac gtggctggat gtacggatca tagatctatc 1920
   tagatgcatt cgcgaggtac cgagctcgaa ttcactggcc gtcgttttac aacgtcgtga 1980
   ctgggaaaac cctggcgtta cccaacttaa tcgccttgca gcacatcccc ctttcgccag 2040
   ctgctagcac catggctcga gcgtggagtt aactcgatgt gagttcaact gtactcttac 2100
   cgagaatgca ccccttttta tagcgctctg aaaagagact gagaatactt tattttctac 2160
   aatcgtataa aatcctagtg atggttacgg taatctttct tcatgggaag ctaaagatat 2220
   tcgatagtta tgcttttggt ttctgtttgg gtgaaactta tgatcagatt atgatgaaac 2280
   ttaaacttgc aagtaatatt tgagtttaaa ctatttttaa tataatcacc taatatgtta 2340
   tgacgtcaat aggcaggaca ttgacccgac gcgtccactc attaaaaata ttattacacc 2400
   ttatataaaa tccatgatat cgttttggtg ttaaaccatt acgtattaac cataacctac 2460
   cccaatgtaa ccttcagtca ccctaatcga tgtttttgta tacatcgatg ttgaccccaa 2520
   caaaagattt ataattaatc ataatcacga acaacaacaa gtcaatgaaa caaataaaca 2580
   agttgtcgat aaaacattca taaatgacac agcaacatac aattcttgca taataaaaat 2640
   ttaaatgaca tcatatttga gaataacaaa tgacattatc cctcgattgt gttttacaag 2700
   tagaattcta cccgtaaagc gagtttagtt ttgaaaaaca aatgacatca tttgtataat 2760
   gacatcatcc cctgattgtg ttttacaagt agaattctat ccgtaaagcg agttcagttt 2820
   tgaaaacaaa tgagtcatac ctaaacacgt taataatctt ctgatatcag cttatgactc 2880
   aagttatgag ccgtgtgcaa aacatgagat aagtttatga catcatccac tgatcgtgcg 2940
   ttacaagtag aattctactc gtaaagccag ttcggttatg agccgtgtgc aaaacatgac 3000
   atcagcttat gactcatact tgattgtgtt ttacgcgtag aattctactc gtaaagcgag 3060
   ttcggttatg agccgtgtgc aaaacatgac atcagcttat gagtcataat taatcgtgcg 3120
   ttacaagtag aattctactc gtaaagcgag ttgaaggatc atatttagtt gcgtttatga 3180
   gataagattg aaagcacgtg taaaatgttt cccgcgcgtt ggcacaacta tttacaatgc 3240
   ggccaagtta taaaagattc taatctgata tgttttaaaa cacctttgcg gcccgagttg 3300
   tttgcgtacg tgactagcga agaagatgtg tggaccgcag aacagatagt aaaacaaaac 3360
   cctagtattg gagcaataat cgatgagctc gtcgacgtag gcctttgaat tccgcgcgct 3420
   tcggaccggg atccaaaaac atcgattagg gtgactgaag gttacattgg ggtaggttat 3480
   ggttaatacg taatggttta acaccaaaac gatatcatgg attttatata aggtgtaata 3540
   atatttttaa tgagtggacg cgtcgggtca atgtcctgcc tattgacgtc ataacatatt 3600
   aggtgattat attaaaaata gtttaaactc aaatattact tgcaagttta agtttcatca 3660
   taatctgatc ataagtttca cccaaacaga aaccaaaagc ataactatcg aatatcttta 3720
   gcttcccatg aagaaagatt accgtaacca tcactaggat tttatacgat tgtagaaaat 3780
   aaagtattct cagtctcttt tcagagcgct ataaaaaggg gtgcattctc ggtaagagta 3840
   cagttgaact cacatcgagt taactccacg ctgcagtctc gagatacgga cctttaattc 3900
   aacccaacac aatatattat agttaaataa gaattattat caaatcattt gtatattaat 3960
   taaaatacta tactgtaaat tacattttat ttacaatcac tcgac 4005
<210> 22
   <211> 3898
   <212> DNA
   <213> Artificial Sequence
<220>
   <223> Recombinant expression cassette
<400> 22
   ttagttgaac tcgaacttct tgtacttgca gttgagcttt tgctcggcga atgtgatggc 60
   gtcggagagt ggcaccagtc cctgcaggat caaggccaag agcttcagca agttgttgtg 120
   cagtgtagtt gactcgcgac ggttgacctt gccgatcacg aacatgtggt gcttgaatct 180
   gttgtacttt tggatgacct ggctcacatc aacgtccttg atttcgccag agatccagta 240
   gaactccttg ttcttcttag cgatggtcag cctttcctcg ttcttgaagg acaacacgat 300
   gaagttgtga cccttaacgt tatcgacgtt ttgagtcaag tactgctcaa cgatgtgcat 360
   gcttccgtct tccttcttga ccttcttgag gttctcggcg ttgttgttag aagcgatgtt 420
   gtcgtggtac ttgtagttgt tgaagagcag gttagcgacg gacgagtact tgtatgtcag 480
   agtgctcttc ttgttcacga tgaggttcag gttatcaacg acgtacttgt tgggagggtt 540
   gtccgggaat tggacggact ccgagtactt gaggatctgg gaaacgtatg gcgagacgaa 600
   gaagttgtta gaggcggttt cgatctcgtt tgattccttg cggctcagca tgatgggcaa 660
   agtgaagagg ttcttgtcct ggtacatctc gtacaacttc gacaagagga atccacactt 720
   acgctcgccg agtgattgca gcaaggtcac gaaggttgtc tgagcgtagt acatatcgag 780
   gttgaagtag gaggtcagag cggccttgaa tgtgtggtgg acgtccacga agtgacactt 840
   cttgcagttt tgagcggcgg tctcatcgtt gcagacgtcc tgtgagtgtg ggatttcgat 900
   gccagtctcc ttaaccaggt tgtagctgat catgaagcgg atcttatcga aggtcacaac 960
   gaacacacgg ttgtcaacca tgtagtagtt gtttgtgtac tcgtagacca cgttagagac 1020
   gtacttggcg aagatgattt cgaaaggctt cacctcggac ttcttaacga cgaacatgta 1080
   gtaaccggtt tcagacatgt gatcggagaa tctgttcgag ttgtagtcgt tatcgtcgaa 1140
   cctcatcagg tagggggcga agtcgtttgt gaagtagtga gtgatctcct gggtggaagc 1200
   cactgtacag atgttagtgt tgtggttgat ggtttgttcc agtgtagcgc atgactggat 1260
   ggtgctcttc ttgtacttag gtctcaactt gatcttgttg aattgaccca caactccctg 1320
   ggagttatcc aggtactcgt ccaacttcct cttggtgcct gtggccgacg gctggttgac 1380
   accagcagag tgttcgaagg actcggcgtg gtaagctgag ctaggagagg gttgttcgac 1440
   cactggctgt tcgagtgact cgctgtagta ggcagaggac acagcttcct ccaggttgtc 1500
   agtggtcttg agcagacact ccaccaaatc gttgtcagtg agcgagttaa ctgaggcgag 1560
   gaagttgcta gcggcagcgg tttcagagtc ggagatgaca gtatcagctc cgtccggggt 1620
   tgggtggttg tagtaagaca agtaatcgtt aggttgcttg tcgcagaact ccgagtatga 1680
   gttgtcgaag ctagcacgag agggagtgct ggcagaggtg taggaagcgt tgaagttgat 1740
   ttgagtcatg gtgacctggt tgttcacgat cttatcgcca cctgtgtcca cctgcagttg 1800
   ctgggcctca gcgcaggctg aagtggcctc acaggagtag gggctggaag cacagttgga 1860
   agtcatgatg ttttcttgga cgttcaggac gtggctggat gtacggatca tagatctatc 1920
   tagatgcatt cgcgaggtac cgagctcgaa ttcactggcc gtcgttttac aacgtcgtga 1980
   ctgggaaaac cctggcgtta cccaacttaa tcgccttgca gcacatcccc ctttcgccag 2040
   ctgctagcac catggctcga gcgtggagtt aactcgatgt gagttcaact gtactcttac 2100
   cgagaatgca ccccttttta tagcgctctg aaaagagact gagaatactt tattttctac 2160
   aatcgtataa aatcctagtg atggttacgg taatctttct tcatgggaag ctaaagatat 2220
   tcgatagtta tgcttttggt ttctgtttgg gtgaaactta tgatcagatt atgatgaaac 2280
   ttaaacttgc aagtaatatt tgagtttaaa ctatttttaa tataatcacc taatatgtta 2340
   tgacgtcaat aggcaggaca ttgacccgac gcgtccactc attaaaaata ttattacacc 2400
   ttatataaaa tccatgatat cgttttggtg ttaaaccatt acgtattaac cataacctac 2460
   cccaatgtaa ccttcagtca ccctaatcga tgtttttgta tacatcgatg ttgaccccaa 2520
   caaaagattt ataattaatc ataatcacga acaacaacaa gtcaatgaaa caaataaaca 2580
   agttgtcgat aaaacattca taaatgacac agcaacatac aattcttgca taataaaaat 2640
   ttaaatgaca tcatatttga gaataacaaa tgacattatc cctcgattgt gttttacaag 2700
   tagaattcta cccgtaaagc gagtttagtt ttgaaaaaca aatgacatca tttgtataat 2760
   gacatcatcc cctgattgtg ttttacaagt agaattctat ccgtaaagcg agttcagttt 2820
   tgaaaacaaa tgagtcatac ctaaacacgt taataatctt ctgatatcag cttatgactc 2880
   aagttatgag ccgtgtgcaa aacatgagat aagtttatga catcatccac tgatcgtgcg 2940
   ttacaagtag aattctactc gtaaagccag ttcggttatg agccgtgtgc aaaacatgac 3000
   atcagcttat gactcatact tgattgtgtt ttacgcgtag aattctactc gtaaagcgag 3060
   ttcggttatg agccgtgtgc aaaacatgac atcagcttat gagtcataat taatcgtgcg 3120
   ttacaagtag aattctactc gtaaagcgag ttgaaggatc atatttagtt gcgtttatga 3180
   gataagattg aaagcacgtg taaaatgttt cccgcgcgtt ggcacaacta tttacaatgc 3240
   ggccaagtta taaaagattc taatctgata tgttttaaaa cacctttgcg gcccgagttg 3300
   tttgcgtacg tgactagcga agaagatgtg tggaccgcag aacagatagt aaaacaaaac 3360
   cctagtattg gagcaataat cgatgagctc gtcgacgtag gcctttgaat tccgcgcgct 3420
   tcggaccggg atcggtacca aattccgttt tgcgacgatg cagagttttt gaacaggctg 3480
   ctcaaacaca tagatccgta cccgctcagt cggatgtatt acaatgcagc caataccatg 3540
   ttttacacga ctatggaaaa ctatgccgtg tccaattgca agttcaacat tgaggattac 3600
   aataacatat ttaaggtgat ggaaaatatt aggaaacaca gcaacaaaaa ttcaaacgac 3660
   caagacgagt taaacatata tttgggagtt cagtcgtcga atgcaaagcg taaaaaatat 3720
   taataaggta aaaattacag ctacataaat tacacaattt aaactgcagt ctggagatac 3780
   ggacctttaa ttcaacccaa cacaatatat tatagttaaa taagaattat tatcaaatca 3840
   tttgtatatt aattaaaata ctatactgta aattacattt tatttacaat cactcgac 3898
<210> 23
   <211> 3179
   <212> DNA
   <213> Artificial Sequence
<220>
   <223> Recombinant expression cassette
<400> 23
   ttagttgaac tcgaacttct tgtacttgca gttgagcttt tgctcggcga atgtgatggc      60
   gtcggagagt ggcaccagtc cctgcaggat caaggccaag agcttcagca agttgttgtg     120
   cagtgtagtt gactcgcgac ggttgacctt gccgatcacg aacatgtggt gcttgaatct     180
   gttgtacttt tggatgacct ggctcacatc aacgtccttg atttcgccag agatccagta     240
   gaactccttg ttcttcttag cgatggtcag cctttcctcg ttcttgaagg acaacacgat     300
   gaagttgtga cccttaacgt tatcgacgtt ttgagtcaag tactgctcaa cgatgtgcat     360
   gcttccgtct tccttcttga ccttcttgag gttctcggcg ttgttgttag aagcgatgtt     420
   gtcgtggtac ttgtagttgt tgaagagcag gttagcgacg gacgagtact tgtatgtcag     480
   agtgctcttc ttgttcacga tgaggttcag gttatcaacg acgtacttgt tgggagggtt     540
   gtccgggaat tggacggact ccgagtactt gaggatctgg gaaacgtatg gcgagacgaa     600
   gaagttgtta gaggcggttt cgatctcgtt tgattccttg cggctcagca tgatgggcaa     660
   agtgaagagg ttcttgtcct ggtacatctc gtacaacttc gacaagagga atccacactt     720
   acgctcgccg agtgattgca gcaaggtcac gaaggttgtc tgagcgtagt acatatcgag     780
   gttgaagtag gaggtcagag cggccttgaa tgtgtggtgg acgtccacga agtgacactt     840
   cttgcagttt tgagcggcgg tctcatcgtt gcagacgtcc tgtgagtgtg ggatttcgat     900
   gccagtctcc ttaaccaggt tgtagctgat catgaagcgg atcttatcga aggtcacaac 960
   gaacacacgg ttgtcaacca tgtagtagtt gtttgtgtac tcgtagacca cgttagagac 1020
   gtacttggcg aagatgattt cgaaaggctt cacctcggac ttcttaacga cgaacatgta 1080
   gtaaccggtt tcagacatgt gatcggagaa tctgttcgag ttgtagtcgt tatcgtcgaa 1140
   cctcatcagg tagggggcga agtcgtttgt gaagtagtga gtgatctcct gggtggaagc 1200
   cactgtacag atgttagtgt tgtggttgat ggtttgttcc agtgtagcgc atgactggat 1260
   ggtgctcttc ttgtacttag gtctcaactt gatcttgttg aattgaccca caactccctg 1320
   ggagttatcc aggtactcgt ccaacttcct cttggtgcct gtggccgacg gctggttgac 1380
   accagcagag tgttcgaagg actcggcgtg gtaagctgag ctaggagagg gttgttcgac 1440
   cactggctgt tcgagtgact cgctgtagta ggcagaggac acagcttcct ccaggttgtc 1500
   agtggtcttg agcagacact ccaccaaatc gttgtcagtg agcgagttaa ctgaggcgag 1560
   gaagttgcta gcggcagcgg tttcagagtc ggagatgaca gtatcagctc cgtccggggt 1620
   tgggtggttg tagtaagaca agtaatcgtt aggttgcttg tcgcagaact ccgagtatga 1680
   gttgtcgaag ctagcacgag agggagtgct ggcagaggtg taggaagcgt tgaagttgat 1740
   ttgagtcatg gtgacctggt tgttcacgat cttatcgcca cctgtgtcca cctgcagttg 1800
   ctgggcctca gcgcaggctg aagtggcctc acaggagtag gggctggaag cacagttgga 1860
   agtcatgatg ttttcttgga cgttcaggac gtggctggat gtacggatca tagatctatt 1920
   gggtcatcta gattcgaaag cggccgcgac tagtgagctc gtcgacgtag gcctttgaat 1980
   tccgcgcgct tcggaccggg atccgcgccc gatggtggga cggtatgaat aatccggaat 2040
   atttataggt ttttttatta caaaactgtt acgaaaacag taaaatactt atttatttgc 2100
   gagatggtta tcattttaat tatctccatg atctattaat attccggagt atacatcgat 2160
   gttgacccca acaaaagatt tataattaat cataatcacg aacaacaaca agtcaatgaa 2220
   acaaataaac aagttgtcga taaaacattc ataaatgaca cagcaacata caattcttgc 2280
   ataataaaaa tttaaatgac atcatatttg agaataacaa atgacattat ccctcgattg 2340
   tgttttacaa gtagaattct acccgtaaag cgagtttagt tttgaaaaac aaatgacatc 2400
   atttgtataa tgacatcatc ccctgattgt gttttacaag tagaattcta tccgtaaagc 2460
   gagttcagtt ttgaaaacaa atgagtcata cctaaacacg ttaataatct tctgatatca 2520
   gcttatgact caagttatga gccgtgtgca aaacatgaga taagtttatg acatcatcca 2580
   ctgatcgtgc gttacaagta gaattctact cgtaaagcca gttcggttat gagccgtgtg 2640
   caaaacatga catcagctta tgactcatac ttgattgtgt tttacgcgta gaattctact 2700
   cgtaaagcga gttcggttat gagccgtgtg caaaacatga catcagctta tgagtcataa 2760
   ttaatcgtgc gttacaagta gaattctact cgtaaagcga gttgaaggat catatttagt 2820
   tgcgtttatg agataagatt gaaagcacgt gtaaaatgtt tcccgcgcgt tggcacaact 2880
   atttacaatg cggccaagtt ataaaagatt ctaatctgat atgttttaaa acacctttgc 2940
   ggcccgagtt gtttgcgtac gtgactagcg aagaagatgt gtggaccgca gaacagatag 3000
   taaaacaaaa ccctagtatt ggagcaataa tcgattccgg aatattaata gatcatggag 3060
   ataattaaaa tgataaccat ctcgcaaata aataagtatt ttactgtttt cgtaacagtt 3120
   ttgtaataaa aaaacctata aatattccgg attattcata ccgtcccacc atcgggcgc 3179
<210> 24
   <211> 3528
   <212> DNA
   <213> Artificial Sequence
<220>
   <223> Recombinant expression cassette
<400> 24
   ttagttgaac tcgaacttct tgtacttgca gttgagcttt tgctcggcga atgtgatggc 60
   gtcggagagt ggcaccagtc cctgcaggat caaggccaag agcttcagca agttgttgtg 120
   cagtgtagtt gactcgcgac ggttgacctt gccgatcacg aacatgtggt gcttgaatct 180
   gttgtacttt tggatgacct ggctcacatc aacgtccttg atttcgccag agatccagta 240
   gaactccttg ttcttcttag cgatggtcag cctttcctcg ttcttgaagg acaacacgat 300
   gaagttgtga cccttaacgt tatcgacgtt ttgagtcaag tactgctcaa cgatgtgcat 360
   gcttccgtct tccttcttga ccttcttgag gttctcggcg ttgttgttag aagcgatgtt 420
   gtcgtggtac ttgtagttgt tgaagagcag gttagcgacg gacgagtact tgtatgtcag 480
   agtgctcttc ttgttcacga tgaggttcag gttatcaacg acgtacttgt tgggagggtt 540
   gtccgggaat tggacggact ccgagtactt gaggatctgg gaaacgtatg gcgagacgaa 600
   gaagttgtta gaggcggttt cgatctcgtt tgattccttg cggctcagca tgatgggcaa 660
   agtgaagagg ttcttgtcct ggtacatctc gtacaacttc gacaagagga atccacactt 720
   acgctcgccg agtgattgca gcaaggtcac gaaggttgtc tgagcgtagt acatatcgag 780
   gttgaagtag gaggtcagag cggccttgaa tgtgtggtgg acgtccacga agtgacactt 840
   cttgcagttt tgagcggcgg tctcatcgtt gcagacgtcc tgtgagtgtg ggatttcgat 900
   gccagtctcc ttaaccaggt tgtagctgat catgaagcgg atcttatcga aggtcacaac 960
   gaacacacgg ttgtcaacca tgtagtagtt gtttgtgtac tcgtagacca cgttagagac 1020
   gtacttggcg aagatgattt cgaaaggctt cacctcggac ttcttaacga cgaacatgta 1080
   gtaaccggtt tcagacatgt gatcggagaa tctgttcgag ttgtagtcgt tatcgtcgaa 1140
   cctcatcagg tagggggcga agtcgtttgt gaagtagtga gtgatctcct gggtggaagc 1200
   cactgtacag atgttagtgt tgtggttgat ggtttgttcc agtgtagcgc atgactggat 1260
   ggtgctcttc ttgtacttag gtctcaactt gatcttgttg aattgaccca caactccctg 1320
   ggagttatcc aggtactcgt ccaacttcct cttggtgcct gtggccgacg gctggttgac 1380
   accagcagag tgttcgaagg actcggcgtg gtaagctgag ctaggagagg gttgttcgac 1440
   cactggctgt tcgagtgact cgctgtagta ggcagaggac acagcttcct ccaggttgtc 1500
   agtggtcttg agcagacact ccaccaaatc gttgtcagtg agcgagttaa ctgaggcgag 1560
   gaagttgcta gcggcagcgg tttcagagtc ggagatgaca gtatcagctc cgtccggggt 1620
   tgggtggttg tagtaagaca agtaatcgtt aggttgcttg tcgcagaact ccgagtatga 1680
   gttgtcgaag ctagcacgag agggagtgct ggcagaggtg taggaagcgt tgaagttgat 1740
   ttgagtcatg gtgacctggt tgttcacgat cttatcgcca cctgtgtcca cctgcagttg 1800
   ctgggcctca gcgcaggctg aagtggcctc acaggagtag gggctggaag cacagttgga 1860
   agtcatgatg ttttcttgga cgttcaggac gtggctggat gtacggatca tagatctatc 1920
   tagatgcatt cgcgaggtac cgagctcgaa ttcactggcc gtcgttttac aacgtcgtga 1980
   ctgggaaaac cctggcgtta cccaacttaa tcgccttgca gcacatcccc ctttcgccag 2040
   ctgctagcac catggctcga gcgtggagtt aactcgatgt gagttcaact gtactcttac 2100
   cgagaatgca ccccttttta tagcgctctg aaaagagact gagaatactt tattttctac 2160
   aatcgtataa aatcctagtg atggttacgg taatctttct tcatgggaag ctaaagatat 2220
   tcgatagtta tgcttttggt ttctgtttgg gtgaaactta tgatcagatt atgatgaaac 2280
   ttaaacttgc aagtaatatt tgagtttaaa ctatttttaa tataatcacc taatatgtta 2340
   tgacgtcaat aggcaggaca ttgacccgac gcgtccactc attaaaaata ttattacacc 2400
   ttatataaaa tccatgatat cgttttggtg ttaaaccatt acgtattaac cataacctac 2460
   cccaatgtaa ccttcagtca ccctaatcga tgtttttgta tacatcgatg ttgaccccaa 2520
   caaaagattt ataattaatc ataatcacga acaacaacaa gtcaatgaaa caaataaaca 2580
   agttgtcgat aaaacattca taaatgacac agcaacatac aattcttgca taataaaaat 2640
   ttaaatgaca tcatatttga gaataacaaa tgacattatc cctcgattgt gttttacaag 2700
   tagaattcta cccgtaaagc gagtttagtt ttgaaaaaca aatgacatca tttgtataat 2760
   gacatcatcc cctgattgtg ttttacaagt agaattctat ccgtaaagcg agttcagttt 2820
   tgaaaacaaa tgagtcatac ctaaacacgt taataatctt ctgatatcag cttatgactc 2880
   aagttatgag ccgtgtgcaa aacatgagat aagtttatga catcatccac tgatcgtgcg 2940
   ttacaagtag aattctactc gtaaagccag ttcggttatg agccgtgtgc aaaacatgac 3000
   atcagcttat gactcatact tgattgtgtt ttacgcgtag aattctactc gtaaagcgag 3060
   ttcggttatg agccgtgtgc aaaacatgac atcagcttat gagtcataat taatcgtgcg 3120
   ttacaagtag aattctactc gtaaagcgag ttgaaggatc atatttagtt gcgtttatga 3180
   gataagattg aaagcacgtg taaaatgttt cccgcgcgtt ggcacaacta tttacaatgc 3240
   ggccaagtta taaaagattc taatctgata tgttttaaaa cacctttgcg gcccgagttg 3300
   tttgcgtacg tgactagcga agaagatgtg tggaccgcag aacagatagt aaaacaaaac 3360
   cctagtattg gagcaataat cgattccgga atattaatag atcatggaga taattaaaat 3420
   gataaccatc tcgcaaataa ataagtattt tactgttttc gtaacagttt tgtaataaaa 3480
   aaacctataa atattccgga ttattcatac cgtcccacca tcgggcgc 3528
<210> 25
   <211> 3291
   <212> DNA
   <213> Artificial Sequence
<220>
   <223> Recombinant expression cassette
<400> 25
   ttagttgaac tcgaacttct tgtacttgca gttgagcttt tgctcggcga atgtgatggc     60
   gtcggagagt ggcaccagtc cctgcaggat caaggccaag agcttcagca agttgttgtg    120
   cagtgtagtt gactcgcgac ggttgacctt gccgatcacg aacatgtggt gcttgaatct    180
   gttgtacttt tggatgacct ggctcacatc aacgtccttg atttcgccag agatccagta    240
   gaactccttg ttcttcttag cgatggtcag cctttcctcg ttcttgaagg acaacacgat    300
   gaagttgtga cccttaacgt tatcgacgtt ttgagtcaag tactgctcaa cgatgtgcat    360
   gcttccgtct tccttcttga ccttcttgag gttctcggcg ttgttgttag aagcgatgtt    420
   gtcgtggtac ttgtagttgt tgaagagcag gttagcgacg gacgagtact tgtatgtcag    480
   agtgctcttc ttgttcacga tgaggttcag gttatcaacg acgtacttgt tgggagggtt    540
   gtccgggaat tggacggact ccgagtactt gaggatctgg gaaacgtatg gcgagacgaa    600
   gaagttgtta gaggcggttt cgatctcgtt tgattccttg cggctcagca tgatgggcaa    660
   agtgaagagg ttcttgtcct ggtacatctc gtacaacttc gacaagagga atccacactt    720
   acgctcgccg agtgattgca gcaaggtcac gaaggttgtc tgagcgtagt acatatcgag    780
   gttgaagtag gaggtcagag cggccttgaa tgtgtggtgg acgtccacga agtgacactt    840
   cttgcagttt tgagcggcgg tctcatcgtt gcagacgtcc tgtgagtgtg ggatttcgat    900
   gccagtctcc ttaaccaggt tgtagctgat catgaagcgg atcttatcga aggtcacaac    960
   gaacacacgg ttgtcaacca tgtagtagtt gtttgtgtac tcgtagacca cgttagagac   1020
   gtacttggcg aagatgattt cgaaaggctt cacctcggac ttcttaacga cgaacatgta   1080
   gtaaccggtt tcagacatgt gatcggagaa tctgttcgag ttgtagtcgt tatcgtcgaa   1140
   cctcatcagg tagggggcga agtcgtttgt gaagtagtga gtgatctcct gggtggaagc 1200
   cactgtacag atgttagtgt tgtggttgat ggtttgttcc agtgtagcgc atgactggat 1260
   ggtgctcttc ttgtacttag gtctcaactt gatcttgttg aattgaccca caactccctg 1320
   ggagttatcc aggtactcgt ccaacttcct cttggtgcct gtggccgacg gctggttgac 1380
   accagcagag tgttcgaagg actcggcgtg gtaagctgag ctaggagagg gttgttcgac 1440
   cactggctgt tcgagtgact cgctgtagta ggcagaggac acagcttcct ccaggttgtc 1500
   agtggtcttg agcagacact ccaccaaatc gttgtcagtg agcgagttaa ctgaggcgag 1560
   gaagttgcta gcggcagcgg tttcagagtc ggagatgaca gtatcagctc cgtccggggt 1620
   tgggtggttg tagtaagaca agtaatcgtt aggttgcttg tcgcagaact ccgagtatga 1680
   gttgtcgaag ctagcacgag agggagtgct ggcagaggtg taggaagcgt tgaagttgat 1740
   ttgagtcatg gtgacctggt tgttcacgat cttatcgcca cctgtgtcca cctgcagttg 1800
   ctgggcctca gcgcaggctg aagtggcctc acaggagtag gggctggaag cacagttgga 1860
   agtcatgatg ttttcttgga cgttcaggac gtggctggat gtacggatca tagatctatt 1920
   gggtcatcta gattcgaaag cggccgcgac tagtgagctc gtcgacgtag gcctttgaat 1980
   tccgcgcgct tcggaccggg atccgcgccc gatggtggga cggtatgaat aatccggaat 2040
   atttataggt ttttttatta caaaactgtt acgaaaacag taaaatactt atttatttgc 2100
   gagatggtta tcattttaat tatctccatg atctattaat attccggagt atacatcgat 2160
   gttgacccca acaaaagatt tataattaat cataatcacg aacaacaaca agtcaatgaa 2220
   acaaataaac aagttgtcga taaaacattc ataaatgaca cagcaacata caattcttgc 2280
   ataataaaaa tttaaatgac atcatatttg agaataacaa atgacattat ccctcgattg 2340
   tgttttacaa gtagaattct acccgtaaag cgagtttagt tttgaaaaac aaatgacatc 2400
   atttgtataa tgacatcatc ccctgattgt gttttacaag tagaattcta tccgtaaagc 2460
   gagttcagtt ttgaaaacaa atgagtcata cctaaacacg ttaataatct tctgatatca 2520
   gcttatgact caagttatga gccgtgtgca aaacatgaga taagtttatg acatcatcca 2580
   ctgatcgtgc gttacaagta gaattctact cgtaaagcca gttcggttat gagccgtgtg 2640
   caaaacatga catcagctta tgactcatac ttgattgtgt tttacgcgta gaattctact 2700
   cgtaaagcga gttcggttat gagccgtgtg caaaacatga catcagctta tgagtcataa 2760
   ttaatcgtgc gttacaagta gaattctact cgtaaagcga gttgaaggat catatttagt 2820
   tgcgtttatg agataagatt gaaagcacgt gtaaaatgtt tcccgcgcgt tggcacaact 2880
   atttacaatg cggccaagtt ataaaagatt ctaatctgat atgttttaaa acacctttgc 2940
   ggcccgagtt gtttgcgtac gtgactagcg aagaagatgt gtggaccgca gaacagatag 3000
   taaaacaaaa ccctagtatt ggagcaataa tcgatgagct catcatggag ataattaaaa 3060
   tgataaccat ctcgcaaata aataagtatt ttactgtttt cgtaacagtt ttgtaataaa 3120
   aaaacctata aatattccgg attattcata ccgtcccacc atcgggcgca tacggacctt 3180
   taattcaacc caacacaata tattatagtt aaataagaat tattatcaaa tcatttgtat 3240
   attaattaaa atactatact gtaaattaca ttttatttac aatcactcga c 3291
<210> 26
   <211> 3640
   <212> DNA
   <213> Artificial Sequence
<220>
   <223> Recombinant expression cassette
<400> 26
   ttagttgaac tcgaacttct tgtacttgca gttgagcttt tgctcggcga atgtgatggc      60
   gtcggagagt ggcaccagtc cctgcaggat caaggccaag agcttcagca agttgttgtg     120
   cagtgtagtt gactcgcgac ggttgacctt gccgatcacg aacatgtggt gcttgaatct     180
   gttgtacttt tggatgacct ggctcacatc aacgtccttg atttcgccag agatccagta     240
   gaactccttg ttcttcttag cgatggtcag cctttcctcg ttcttgaagg acaacacgat     300
   gaagttgtga cccttaacgt tatcgacgtt ttgagtcaag tactgctcaa cgatgtgcat     360
   gcttccgtct tccttcttga ccttcttgag gttctcggcg ttgttgttag aagcgatgtt     420
   gtcgtggtac ttgtagttgt tgaagagcag gttagcgacg gacgagtact tgtatgtcag     480
   agtgctcttc ttgttcacga tgaggttcag gttatcaacg acgtacttgt tgggagggtt     540
   gtccgggaat tggacggact ccgagtactt gaggatctgg gaaacgtatg gcgagacgaa     600
   gaagttgtta gaggcggttt cgatctcgtt tgattccttg cggctcagca tgatgggcaa     660
   agtgaagagg ttcttgtcct ggtacatctc gtacaacttc gacaagagga atccacactt     720
   acgctcgccg agtgattgca gcaaggtcac gaaggttgtc tgagcgtagt acatatcgag     780
   gttgaagtag gaggtcagag cggccttgaa tgtgtggtgg acgtccacga agtgacactt     840
   cttgcagttt tgagcggcgg tctcatcgtt gcagacgtcc tgtgagtgtg ggatttcgat     900
   gccagtctcc ttaaccaggt tgtagctgat catgaagcgg atcttatcga aggtcacaac     960
   gaacacacgg ttgtcaacca tgtagtagtt gtttgtgtac tcgtagacca cgttagagac    1020
   gtacttggcg aagatgattt cgaaaggctt cacctcggac ttcttaacga cgaacatgta    1080
   gtaaccggtt tcagacatgt gatcggagaa tctgttcgag ttgtagtcgt tatcgtcgaa    1140
   cctcatcagg tagggggcga agtcgtttgt gaagtagtga gtgatctcct gggtggaagc    1200
   cactgtacag atgttagtgt tgtggttgat ggtttgttcc agtgtagcgc atgactggat    1260
   ggtgctcttc ttgtacttag gtctcaactt gatcttgttg aattgaccca caactccctg    1320
   ggagttatcc aggtactcgt ccaacttcct cttggtgcct gtggccgacg gctggttgac 1380
   accagcagag tgttcgaagg actcggcgtg gtaagctgag ctaggagagg gttgttcgac 1440
   cactggctgt tcgagtgact cgctgtagta ggcagaggac acagcttcct ccaggttgtc 1500
   agtggtcttg agcagacact ccaccaaatc gttgtcagtg agcgagttaa ctgaggcgag 1560
   gaagttgcta gcggcagcgg tttcagagtc ggagatgaca gtatcagctc cgtccggggt 1620
   tgggtggttg tagtaagaca agtaatcgtt aggttgcttg tcgcagaact ccgagtatga 1680
   gttgtcgaag ctagcacgag agggagtgct ggcagaggtg taggaagcgt tgaagttgat 1740
   ttgagtcatg gtgacctggt tgttcacgat cttatcgcca cctgtgtcca cctgcagttg 1800
   ctgggcctca gcgcaggctg aagtggcctc acaggagtag gggctggaag cacagttgga 1860
   agtcatgatg ttttcttgga cgttcaggac gtggctggat gtacggatca tagatctatc 1920
   tagatgcatt cgcgaggtac cgagctcgaa ttcactggcc gtcgttttac aacgtcgtga 1980
   ctgggaaaac cctggcgtta cccaacttaa tcgccttgca gcacatcccc ctttcgccag 2040
   ctgctagcac catggctcga gcgtggagtt aactcgatgt gagttcaact gtactcttac 2100
   cgagaatgca ccccttttta tagcgctctg aaaagagact gagaatactt tattttctac 2160
   aatcgtataa aatcctagtg atggttacgg taatctttct tcatgggaag ctaaagatat 2220
   tcgatagtta tgcttttggt ttctgtttgg gtgaaactta tgatcagatt atgatgaaac 2280
   ttaaacttgc aagtaatatt tgagtttaaa ctatttttaa tataatcacc taatatgtta 2340
   tgacgtcaat aggcaggaca ttgacccgac gcgtccactc attaaaaata ttattacacc 2400
   ttatataaaa tccatgatat cgttttggtg ttaaaccatt acgtattaac cataacctac 2460
   cccaatgtaa ccttcagtca ccctaatcga tgtttttgta tacatcgatg ttgaccccaa 2520
   caaaagattt ataattaatc ataatcacga acaacaacaa gtcaatgaaa caaataaaca 2580
   agttgtcgat aaaacattca taaatgacac agcaacatac aattcttgca taataaaaat 2640
   ttaaatgaca tcatatttga gaataacaaa tgacattatc cctcgattgt gttttacaag 2700
   tagaattcta cccgtaaagc gagtttagtt ttgaaaaaca aatgacatca tttgtataat 2760
   gacatcatcc cctgattgtg ttttacaagt agaattctat ccgtaaagcg agttcagttt 2820
   tgaaaacaaa tgagtcatac ctaaacacgt taataatctt ctgatatcag cttatgactc 2880
   aagttatgag ccgtgtgcaa aacatgagat aagtttatga catcatccac tgatcgtgcg 2940
   ttacaagtag aattctactc gtaaagccag ttcggttatg agccgtgtgc aaaacatgac 3000
   atcagcttat gactcatact tgattgtgtt ttacgcgtag aattctactc gtaaagcgag 3060
   ttcggttatg agccgtgtgc aaaacatgac atcagcttat gagtcataat taatcgtgcg 3120
   ttacaagtag aattctactc gtaaagcgag ttgaaggatc atatttagtt gcgtttatga 3180
   gataagattg aaagcacgtg taaaatgttt cccgcgcgtt ggcacaacta tttacaatgc 3240
   ggccaagtta taaaagattc taatctgata tgttttaaaa cacctttgcg gcccgagttg 3300
   tttgcgtacg tgactagcga agaagatgtg tggaccgcag aacagatagt aaaacaaaac 3360
   cctagtattg gagcaataat cgatgagctc atcatggaga taattaaaat gataaccatc 3420
   tcgcaaataa ataagtattt tactgttttc gtaacagttt tgtaataaaa aaacctataa 3480
   atattccgga ttattcatac cgtcccacca tcgggcgcat acggaccttt aattcaaccc 3540
   aacacaatat attatagtta aataagaatt attatcaaat catttgtata ttaattaaaa 3600
   tactatactg taaattacat tttatttaca atcactcgac 3640
<210> 27
   <211> 881
   <212> DNA
   <213> Autographa californica nucleopolyhedrovirus
<400> 27
   atcgatgttg accccaacaa aagatttata attaatcata atcacgaaca acaacaagtc 60
   aatgaaacaa ataaacaagt tgtcgataaa acattcataa atgacacagc aacatacaat 120
   tcttgcataa taaaaattta aatgacatca tatttgagaa taacaaatga cattatccct 180
   cgattgtgtt ttacaagtag aattctaccc gtaaagcgag tttagttttg aaaaacaaat 240
   gacatcattt gtataatgac atcatcccct gattgtgttt tacaagtaga attctatccg 300
   taaagcgagt tcagttttga aaacaaatga gtcataccta aacacgttaa taatcttctg 360
   atatcagctt atgactcaag ttatgagccg tgtgcaaaac atgagataag tttatgacat 420
   catccactga tcgtgcgtta caagtagaat tctactcgta aagccagttc ggttatgagc 480
   cgtgtgcaaa acatgacatc agcttatgac tcatacttga ttgtgtttta cgcgtagaat 540
   tctactcgta aagcgagttc ggttatgagc cgtgtgcaaa acatgacatc agcttatgag 600
   tcataattaa tcgtgcgtta caagtagaat tctactcgta aagcgagttg aaggatcata 660
   tttagttgcg tttatgagat aagattgaaa gcacgtgtaa aatgtttccc gcgcgttggc 720
   acaactattt acaatgcggc caagttataa aagattctaa tctgatatgt tttaaaacac 780
   ctttgcggcc cgagttgttt gcgtacgtga ctagcgaaga agatgtgtgg accgcagaac 840
   agatagtaaa acaaaaccct agtattggag caataatcga t 881
<210> 28
   <211> 2124
   <212> DNA
   <213> Artificial Sequence
<220>
   <223> Recombinant DNA construct fusing the Ac-ie-01 cDNA to the polh promoter
<400> 28
   atcatggaga taattaaaat gataaccatc tcgcaaataa ataagtattt tactgttttc 60
   gtaacagttt tgtaataaaa aaacctataa atattccgga ttattcatac cgtcccacca 120
   tcgggcgcgg atcccggtcc gaagcgcgcg gaattcaaag gcctacgtcg acgagctcac 180
   tagtcgcggc cgctttcgaa tctagataga tctatgatcc gtacatccag ccacgtcctg 240
   aacgtccaag aaaacatcat gacttccaac tgtgcttcca gcccctactc ctgtgaggcc 300
   acttcagcct gcgctgaggc ccagcaactg caggtggaca caggtggcga taagatcgtg 360
   aacaaccagg tcaccatgac tcaaatcaac ttcaacgctt cctacacctc tgccagcact 420
   ccctctcgtg ctagcttcga caactcatac tcggagttct gcgacaagca acctaacgat 480
   tacttgtctt actacaacca cccaaccccg gacggagctg atactgtcat ctccgactct 540
   gaaaccgctg ccgctagcaa cttcctcgcc tcagttaact cgctcactga caacgatttg 600
   gtggagtgtc tgctcaagac cactgacaac ctggaggaag ctgtgtcctc tgcctactac 660
   agcgagtcac tcgaacagcc agtggtcgaa caaccctctc ctagctcagc ttaccacgcc 720
   gagtccttcg aacactctgc tggtgtcaac cagccgtcgg ccacaggcac caagaggaag 780
   ttggacgagt acctggataa ctcccaggga gttgtgggtc aattcaacaa gatcaagttg 840
   agacctaagt acaagaagag caccatccag tcatgcgcta cactggaaca aaccatcaac 900
   cacaacacta acatctgtac agtggcttcc acccaggaga tcactcacta cttcacaaac 960
   gacttcgccc cctacctgat gaggttcgac gataacgact acaactcgaa cagattctcc 1020
   gatcacatgt ctgaaaccgg ttactacatg ttcgtcgtta agaagtccga ggtgaagcct 1080
   ttcgaaatca tcttcgccaa gtacgtctct aacgtggtct acgagtacac aaacaactac 1140
   tacatggttg acaaccgtgt gttcgttgtg accttcgata agatccgctt catgatcagc 1200
   tacaacctgg ttaaggagac tggcatcgaa atcccacact cacaggacgt ctgcaacgat 1260
   gagaccgccg ctcaaaactg caagaagtgt cacttcgtgg acgtccacca cacattcaag 1320
   gccgctctga cctcctactt caacctcgat atgtactacg ctcagacaac cttcgtgacc 1380
   ttgctgcaat cactcggcga gcgtaagtgt ggattcctct tgtcgaagtt gtacgagatg 1440
   taccaggaca agaacctctt cactttgccc atcatgctga gccgcaagga atcaaacgag 1500
   atcgaaaccg cctctaacaa cttcttcgtc tcgccatacg tttcccagat cctcaagtac 1560
   tcggagtccg tccaattccc ggacaaccct cccaacaagt acgtcgttga taacctgaac 1620
   ctcatcgtga acaagaagag cactctgaca tacaagtact cgtccgtcgc taacctgctc 1680
   ttcaacaact acaagtacca cgacaacatc gcttctaaca acaacgccga gaacctcaag 1740
   aaggtcaaga aggaagacgg aagcatgcac atcgttgagc agtacttgac tcaaaacgtc 1800
   gataacgtta agggtcacaa cttcatcgtg ttgtccttca agaacgagga aaggctgacc 1860
   atcgctaaga agaacaagga gttctactgg atctctggcg aaatcaagga cgttgatgtg 1920
   agccaggtca tccaaaagta caacagattc aagcaccaca tgttcgtgat cggcaaggtc 1980
   aaccgtcgcg agtcaactac actgcacaac aacttgctga agctcttggc cttgatcctg 2040
   cagggactgg tgccactctc cgacgccatc acattcgccg agcaaaagct caactgcaag 2100
   tacaagaagt tcgagttcaa ctaa 2124
<210> 29
   <211> 911
   <212> DNA
   <213> Artificial Sequence
<220>
   <223> Recombinant DNA construct fusing the GFP cDNA to the polh promoter
<400> 29
   atcatggaga taattaaaat gataaccatc tcgcaaataa ataagtattt tactgttttc 60
   gtaacagttt tgtaataaaa aaacctataa atattccgga ttattcatac cgtcccacca 120
   tcgggcgcgg atccaaggcc actagtgcgg ccgctctgca gtctcgagca tgcggtacca 180
   agcttgaatt catggtgagc aagggcgagg agctgttcac cggggtggtg cccatcctgg 240
   tcgagctgga cggcgacgta aacggccaca agttcagcgt gtccggcgag ggcgagggcg 300
   atgccaccta cggcaagctg accctgaagt tcatctgcac caccggcaag ctgcccgtgc 360
   cctggcccac cctcgtgacc accctgacct acggcgtgca gtgcttcagc cgctaccccg 420
   accacatgaa gcagcacgac ttcttcaagt ccgccatgcc cgaaggctac gtccaggagc 480
   gcaccatctt cttcaaggac gacggcaact acaagacccg cgccgaggtg aagttcgagg 540
   gcgacaccct ggtgaaccgc atcgagctga agggcatcga cttcaaggag gacggcaaca 600
   tcctggggca caagctggag tacaactaca acagccacaa cgtctatatc atggccgaca 660
   agcagaagaa cggcatcatg gtgaacttca agatccgcca caacatcgag gacggcagcg 720
   tgcagctcgc cgaccactac cagcagaaca cccccatcgg cgacggcccc gtgctgctgc 780
   ccgacaacca ctacctgagc acccagtccg ccctgagcaa agaccccaac gagaagcgcg 840
   atcacatggt cctgctggag ttcgtgaccg ccgccgggat cactctcggc atggacgagc 900
   tgtacaagta a 911